(19) Europäisches Patentamt
European Patent Office
Office européen des brevets

(11) EP 3 318 573 A1

(12) EUROPEAN PATENT APPLICATION

(43) Date of publication:
**09.05.2018 Bulletin 2018/19**

(21) Application number: **17202575.1**

(22) Date of filing: **23.12.2009**

(51) Int Cl.:
*C07K 14/31* (2006.01)
*C07K 16/12* (2006.01)
*A61K 39/395* (2006.01)
*C07K 16/00* (2006.01)
*C07K 16/32* (2006.01)

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **23.12.2008 US 140565 P**

(62) Document number(s) of the earlier application(s) in accordance with Art. 76 EPC:
**09796273.2 / 2 382 234**

(71) Applicant: **F. Hoffmann-La Roche AG**
**4070 Basel (CH)**

(72) Inventors:
- **Lowman, Henry B.**
  **South San Francisco, CA 94080 (US)**
- **Yeung, Yik Andy**
  **South San Francisco, CA 94080 (US)**

(74) Representative: **Brodbeck, Michel**
**F. Hoffmann-La Roche AG**
**Patent Department**
**Grenzacherstrasse 124**
**4070 Basel (CH)**

Remarks:
This application was filed on 20.11.2017 as a divisional application to the application mentioned under INID code 62.

# (54) MMUNOGLOBULIN VARIANTS WITH ALTERED BINDING TO PROTEIN A

(57) Variant immunoglobulins with one or more amino acid modifications in the $V_H$ region that have altered binding to *Staphylococcus aureus* protein A, and methods of using the same are provided.

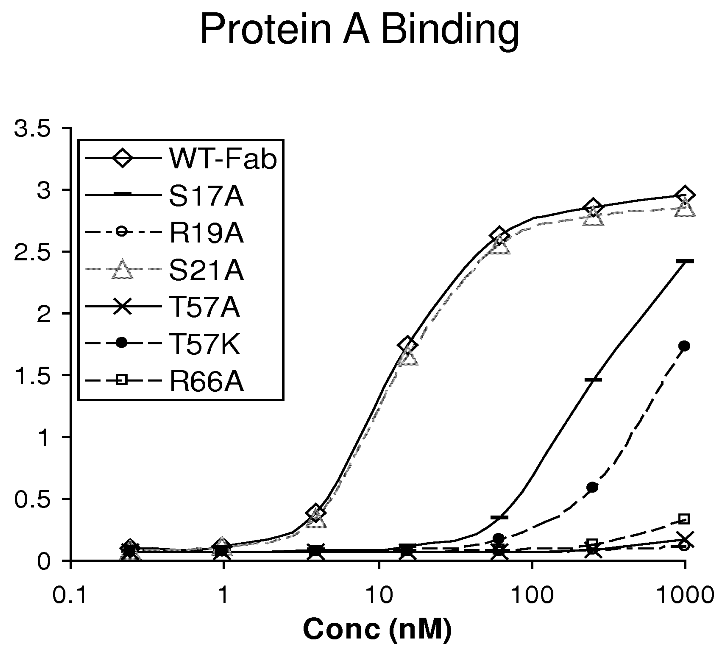


Figure 3

EP 3 318 573 A1

**Description**

## RELATED APPLICATION

This application is a non-provisional application filed under 37 CFR 1.53(b)(1), claiming priority under 35 USC 119(e) to provisional application number 61/140565 filed December 23, 2008, the contents of which are incorporated herein by reference.

## FIELD OF THE INVENTION

The present invention relates generally to the field of molecular biology. More specifically, the present invention relates to IgG immunoglobulin variants with altered biological properties and methods of using the same.

## BACKGROUND OF THE INVENTION

Over the years the use of immunoglobulins as therapeutic agents has increased dramatically. Immunoglobulin (Ig) molecules which constitute an important part of the immune system are of great interest because they (1) react with a diverse family of ligands, (2) possess different effector functions and (3) are of great biological importance. Today uses of antibody based drugs include treatment of cancer, autoimmune diseases as well as various systemic and infectious diseases. Also, immunoglobulins are useful as *in vivo* diagnostic tools, for example, in diagnostic imaging procedures.

IgG is the most prevalent immunoglobulin class in humans and other mammals and is utilized in various types of immunotherapies and diagnostic procedures. Human $IgG_1$ is the most commonly used antibody for therapeutic purposes. One area of active research is antibodies against pathogens, including *Staphylococcus aureus.* Despite its potential, one of the problems with immunoglobulin therapy targeting *S. aureus* has been the binding of antibodies to *S. aureus* protein A. The binding of IgG to protein A has been studied and positions involved in the binding to both protein A and FcRn have been identified (*see, e.g.,* Riechmann & Davies, J. Biomolecular NMR 6:141-52 (1995), Artandi et al., Proc. Natl. Acad. Sci. USA 89:94-98 (1992), WO 93/22332). It would be advantageous to have modified immunoglobulins that exhibit altered binding to protein A. The present invention addresses these and other needs, as will be apparent upon review of the following disclosure.

## SUMMARY OF THE INVENTION

The invention provides novel IgG variants and uses thereof. A number of IgG variants are provided in the invention.

In one aspect, the invention provides variant IgG comprising a human IgG $V_H$ region comprising one or more amino acid substitutions relative to a wild-type human IgG $V_H$ region at one or more of amino acid residues 17, 19, 57, 66, 70, 79, 81, 82a, 82b, numbered according to the EU index as in Kabat, wherein the variant IgG has altered binding to *Staphylococcus aureus* protein A. In some embodiments, the variant IgG has increased binding to protein A, *e.g.* one with a human IgG $V_H$ region comprising one or more amino acid substitutions relative to a wild-type IgG $V_H$ region at one or more of amino acid residues 70, 79, and 82b (e.g. S70A, Y79A, or S82bA). In some embodiments, the variant IgG has decreased binding to protein A, e.g. one with a human IgG $V_H$ region comprising one or more amino acid substitutions relative to a wild-type IgG $V_H$ region at one or more of amino acid residues 17, 19, 57, 66, 81, and 82a (e.g. S17A, R19A, T57A, T57K, R66A, Q81A, or N82aA). In some embodiments, the variant IgG is a human or humanized IgG. In some embodiments, the variant IgG is IgG1, IgG2, IgG3 or IgG4. In some embodiments, the variant IgG binds to a *Staphylococcus aureus* protein other than protein A. In some embodiments, the invention provides a pharmaceutical composition comprising a variant IgG of the invention and a pharmaceutically acceptable carrier. In some embodiments, the invention provides a kit comprising a variant IgG of the invention in a container, and instructions for use.

Other features and advantages of the invention will be apparent from the following Detailed Description, the drawings, and the claims.

Any embodiment described herein or any combination thereof applies to any and all variant IgGs and methods of the invention described herein.

## BRIEF DESCRIPTION OF THE FIGURES

Figures 1 and 2 show ELISAs of the binding of variant anti-Her2 Fabs to Her2.

Figures 3 and 4 show ELISAs of the binding of variant anti-Her2 Fabs to protein A.

## DETAILED DESCRIPTION OF THE INVENTION

The present invention relates to novel variants of IgG domains, including those found in antibodies and fusion proteins, that have altered binding to *S. aureus* protein A. These variants comprise a human IgG $V_H$ region, or fragment thereof that binds to protein A, that contains one or more amino acid modifications relative to a wild-type human $V_H$ region which modifications alter its affinity for protein A.

The techniques and procedures described or referenced herein are generally well understood and commonly employed using conventional methodology by those skilled in the art, such as, for example, the widely utilized methodologies described in Sambrook et al., Molecular Cloning: A Laboratory Manual 3rd. edition (2001) Cold Spring Harbor Laboratory Press, Cold Spring Harbor, N.Y. CURRENT PROTOCOLS IN MOLECULAR BIOLOGY (F. M. Ausubel, et al. eds., (2003)); the series METHODS IN ENZYMOLOGY (Academic Press, Inc.): PCR 2: A PRACTICAL APPROACH (M. J. MacPherson, B. D. Hames and G. R. Taylor eds. (1995)), Harlow and Lane, eds. (1988) ANTIBODIES, A LABORATORY MANUAL, and ANIMAL CELL CULTURE (R. I. Freshney, ed. (1987)); Oligonucleotide Synthesis (M. J. Gait, ed., 1984); Methods in Molecular Biology, Humana Press; Cell Biology: A Laboratory Notebook (J. E. Cellis, ed., 1998) Academic Press; Animal Cell Culture (R. I. Freshney), ed., 1987); Introduction to Cell and Tissue Culture (J. P. Mather and P. E. Roberts, 1998) Plenum Press; Cell and Tissue Culture: Laboratory Procedures (A. Doyle, J. B. Griffiths, and D. G. Newell, eds., 1993-8) J. Wiley and Sons; Handbook of Experimental Immunology (D. M. Weir and C. C. Blackwell, eds.); Gene Transfer Vectors for Mammalian Cells (J. M. Miller and M. P. Calos, eds., 1987); PCR: The Polymerase Chain Reaction, (Mullis et al., eds., 1994); Current Protocols in Immunology (J. E. Coligan et al., eds., 1991); Short Protocols in Molecular Biology (Wiley and Sons, 1999); Immunobiology (C. A. Janeway and P. Travers, 1997); Antibodies (P. Finch, 1997); Antibodies: A Practical Approach (D. Catty., ed., IRL Press, 1988-1989); Monoclonal Antibodies: A Practical Approach (P. Shepherd and C. Dean, eds., Oxford University Press, 2000); Using Antibodies: A Laboratory Manual (E. Harlow and D. Lane (Cold Spring Harbor Laboratory Press, 1999); The Antibodies (M. Zanetti and J. D. Capra, eds., Harwood Academic Publishers, 1995); and Cancer: Principles and Practice of Oncology (V. T. DeVita et al., eds., J.B. Lippincott Company, 1993).

Unless defined otherwise, technical and scientific terms used herein have the same meaning as commonly understood by one of ordinary skill in the art to which this invention belongs. Singleton et al., Dictionary of Microbiology and Molecular Biology 2nd ed., J. Wiley & Sons (New York, N.Y. 1994), and March, Advanced Organic Chemistry Reactions, Mechanisms and Structure 4th ed., John Wiley & Sons (New York, N.Y. 1992), provide one skilled in the art with a general guide to many of the terms used in the present application. All references cited herein, including patent applications and publications, are incorporated by reference in their entirety.

### Definitions

For purposes of interpreting this specification, the following definitions will apply and whenever appropriate, terms used in the singular will also include the plural and vice versa. It is to be understood that the terminology used herein is for the purpose of describing particular embodiments only, and is not intended to be limiting. In the event that any definition set forth below conflicts with any document incorporated herein by reference, the definition set forth below shall control.

Throughout the present specification and claims, the numbering of the residues in an immunoglobulin heavy chain is that of the EU index as in Kabat et al., Sequences of Proteins of Immunological Interest, 5th Ed. Public Health Service, National Institutes of Health, Bethesda, Md. (1991). The "EU index as in Kabat" refers to the residue numbering of the human $IgG_1$ EU antibody.

By "parent polypeptide" or "wild-type polypeptide" as used herein is meant an unmodified polypeptide, a naturally occurring polypeptide, or an engineered modified version of a naturally occurring polypeptide which lacks one or more of the amino acid modifications disclosed herein and which differs in protein A binding compared to variant protein as herein disclosed. The parent polypeptide may comprise a native sequence $V_H$ region or a $V_H$ region with pre-existing amino acid sequence modifications (such as additions, deletions and/or substitutions). The parent polypeptide may also comprise non-natural amino acids as described below. Parent polypeptide may refer to the polypeptide itself, compositions that comprise the parent polypeptide, or the amino acid sequence that encodes it. Parent polypeptide, includes, without limitation, parent immunoglobulin, wild-type immunoglobulin, parent antibody and wild-type antibody.

Accordingly, by "parent immunoglobulin," "parent IgG," "wild-type immunoglobulin" or "wild-type IgG" as used herein is meant an unmodified immunoglobulin, a naturally occurring immunoglobulin, or an engineered modified version of a naturally occurring immunoglobulin which lacks one or more of the amino acid modifications disclosed herein and which differs in protein A binding compared to variant IgG as herein disclosed. The parent immunoglobulin may comprise a native sequence $V_H$ region or a $V_H$ region with pre-existing amino acid sequence modifications (such as additions, deletions and/or substitutions). The parent immunoglobulin may also comprise non-natural amino acids as described below. Parent immunoglobulin may refer to the immunoglobulin itself, compositions that comprise the parent immunoglobulin, or the amino acid sequence that encodes it.

By "parent antibody" or "wild-type antibody" as used herein is meant an unmodified antibody, a naturally occurring antibody, or an engineered modified version of a naturally occurring antibody which lacks one or more of the amino acid modifications disclosed herein and which differs in protein A binding compared to variant IgG as herein disclosed. The parent antibody may comprise a native sequence $V_H$ region or a $V_H$ region with pre-existing amino acid sequence modifications (such as additions, deletions and/or substitutions). The parent antibody may also comprise non-natural amino acids as described below. Parent antibody may refer to the antibody itself, compositions that comprise the parent antibody, or the amino acid sequence that encodes it.

By "variant," "variant protein" or "protein variant" as used herein is meant a protein that differs from that of a parent protein by virtue of at least one amino acid modification. Protein variant may refer to the protein itself, a composition comprising the protein, or the amino sequence that encodes it. In certain embodiments, the protein variant has at least one amino acid modification compared to the parent polypeptide, e.g. from about one to about ten amino acid modifications. The protein variant sequence herein will preferably possess at least about 80% homology with a parent protein sequence, and most preferably at least about 90% homology, more preferably at least about 95% homology. Protein variants may also comprise non-natural amino acids, as defined below. The term "protein variant" includes immunoglobulin variant and antibody variant as described herein.

The term "immunoglobulin variant," "variant immunoglobulin," "variant IgG" or "IgG variant" as used herein is meant an immunoglobulin sequence that differs from that of a parent or wild-type immunoglobulin sequence by virtue of at least one amino acid modification.

By "antibody variant" or "variant antibody" as used herein is meant an antibody that differs from a parent antibody by virtue of at least one amino acid modification. In certain embodiments, the variant antibody has one or more amino acid modifications in the $V_H$ region relative to wild-type antibody. In certain embodiments, a variant antibody is a variant IgG.

By "position" as used herein is meant a location in the sequence of a protein. Positions may be numbered sequentially, or according to an established format, for example the EU index as in Kabat.

An "amino acid modification" refers to a change in the amino acid sequence of a predetermined amino acid sequence. Exemplary modifications include an amino acid substitution, insertion and/or deletion. In certain embodiments, the amino acid modification is a substitution.

An "amino acid modification at" a specified position, e.g. of the Fc region, refers to the substitution or deletion of the specified residue, or the insertion of at least one amino acid residue adjacent the specified residue. By insertion "adjacent" to a specified residue is meant insertion within one to two residues thereof. The insertion may be N-terminal or C-terminal to the specified residue.

An "amino acid substitution" refers to the replacement of at least one existing amino acid residue in a predetermined amino acid sequence with another different "replacement" amino acid residue. The replacement residue or residues may be "naturally occurring amino acid residues" (*i.e.* encoded by the genetic code) and selected from the group consisting of: alanine (Ala); arginine (Arg); asparagine (Asn); aspartic acid (Asp); cysteine (Cys); glutamine (Gln); glutamic acid (Glu); glycine (Gly); histidine (His); isoleucine (Ile): leucine (Leu); lysine (Lys); methionine (Met); phenylalanine (Phe); proline (Pro); serine (Ser); threonine (Thr); tryptophan (Trp); tyrosine (Tyr); and valine (Val). Substitution with one or more non-naturally occurring amino acid residues is also encompassed by the definition of an amino acid substitution herein.

A "non-naturally occurring amino acid residue" refers to a residue, other than those naturally occurring amino acid residues listed above, which is able to covalently bind adjacent amino acid residues(s) in a polypeptide chain. Examples of non-naturally occurring amino acid residues include norleucine, ornithine, norvaline, homoserine and other amino acid residue analogues such as those described in Ellman et al. Meth. Enzym. 202:301-336 (1991); US Patent No. 6,586,207; WO 98/48032; WO 03/073238; U.S. Publication No. 2004-0214988A1; WO 05135727A2; WO 05/74524A2; J. W. Chin et al., (2002), Journal of the American Chemical Society 124:9026-9027; J. W. Chin, & P. G. Schultz, (2002), ChemBioChem 11:1135-1137; and J. W. Chin, et al., (2002), PICAS United States of America 99:11020-11024.

In certain embodiments, the terms "decrease", "decrease protein A binding", "reduce", or "reduce protein A binding" refers to an overall decrease of 25%, 30%, 40%, 50%, 60%, 70%, 80%, 85%, 90%, 95%, 97%, or 99% in the protein A binding of a variant IgG of the invention detected by standard art known methods such as those described herein, as compared to a wild-type IgG or an IgG having the wild-type human IgG Fc region. In certain embodiments these terms alternatively may refer to an overall decrease of 10-fold (*i.e.* 1 log), 100-fold (2 logs), 1,000-fold (or 3 logs), 10,000-fold (or 4 logs), or 100,000-fold (or 5 logs). Similarly, the terms "increase", "increase protein A binding" and the like refer to an overall increase of 2-fold, 3-fold, 4-fold, 5-fold, 10-fold, 100-fold, or 1,000-fold in the protein A binding of a variant IgG of the invention detected by standard art known methods such as those described herein, as compared to a wild-type IgG or an IgG having the wild-type human IgG Fc region.

The term "antibody" herein is used in the broadest sense and specifically covers monoclonal antibodies (including full length monoclonal antibodies), polyclonal antibodies, multispecific antibodies (*e.g.*, bispecific antibodies), and antibody fragments so long as they exhibit the desired biological activity.

An "isolated" antibody or other polypeptide is one which has been identified and separated and/or recovered from a component of its natural environment. Contaminant components of its natural environment are materials which would interfere with research, diagnostic or therapeutic uses for the antibody, and may include enzymes, hormones, and other proteinaceous or nonproteinaceous solutes. In some embodiments, an antibody or other polypeptide is purified (1) to greater than 95% by weight of antibody or other polypeptide as determined by, for example, the Lowry method, and in some embodiments, to greater than 99% by weight; (2) to a degree sufficient to obtain at least 15 residues of N-terminal or internal amino acid sequence by use of, for example, a spinning cup sequenator, or (3) to homogeneity by SDS-PAGE under reducing or nonreducing conditions using, for example, Coomassie blue or silver stain. Isolated antibody or other polypeptide includes it *in situ* within recombinant cells since at least one component of the its natural environment will not be present. Ordinarily, however, isolated antibody or other polypeptide will be prepared by at least one purification step.

"Native antibodies" are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies among the heavy chains of different immunoglobulin isotypes. Each heavy and light chain also has regularly spaced intrachain disulfide bridges. Each heavy chain has at one end a variable domain ($V_H$) followed by a number of constant domains. Each light chain has a variable domain at one end ($V_L$) and a constant domain at its other end; the constant domain of the light chain is aligned with the first constant domain of the heavy chain, and the light chain variable domain is aligned with the variable domain of the heavy chain. Particular amino acid residues are believed to form an interface between the light chain and heavy chain variable domains.

The term "constant domain" refers to the portion of an immunoglobulin molecule having a more conserved amino acid sequence relative to the other portion of the immunoglobulin, the variable domain, which contains the antigen binding site. The constant domain contains the $C_H1$, $C_H2$ and $C_H3$ domains of the heavy chain and the $C_HL$ domain of the light chain.

The "variable region" or "variable domain" of an antibody refers to the amino-terminal domains of the heavy or light chain of the antibody. The variable domain of the heavy chain may be referred to as "$V_H$." The variable domain of the light chain may be referred to as "$V_L$." These domains are generally the most variable parts of an antibody and contain the antigen-binding sites.

The term "variable" refers to the fact that certain portions of the variable domains differ extensively in sequence among antibodies and are used in the binding and specificity of each particular antibody for its particular antigen. However, the variability is not evenly distributed throughout the variable domains of antibodies. It is concentrated in three segments called hypervariable regions (HVRs) both in the light-chain and the heavy-chain variable domains. The more highly conserved portions of variable domains are called the framework regions (FR). The variable domains of native heavy and light chains each comprise four FR regions, largely adopting a beta-sheet configuration, connected by three HVRs, which form loops connecting, and in some cases forming part of, the beta-sheet structure. The HVRs in each chain are held together in close proximity by the FR regions and, with the HVRs from the other chain, contribute to the formation of the antigen-binding site of antibodies (see Kabat et al., Sequences of Proteins of Immunological Interest, Fifth Edition, National Institute of Health, Bethesda, MD (1991)). The constant domains are not involved directly in the binding of an antibody to an antigen, but exhibit various effector functions, such as participation of the antibody in antibody-dependent cellular toxicity.

The "light chains" of antibodies (immunoglobulins) from any vertebrate species can be assigned to one of two clearly distinct types, called kappa ($\kappa$) and lambda ($\lambda$), based on the amino acid sequences of their constant domains.

The term IgG "isotype" or "subclass" as used herein is meant any of the subclasses of immunoglobulins defined by the chemical and antigenic characteristics of their constant regions.

Depending on the amino acid sequences of the constant domains of their heavy chains, antibodies (immunoglobulins) can be assigned to different classes. There are five major classes of immunoglobulins: IgA, IgD, IgE, IgG, and IgM, and several of these may be further divided into subclasses (isotypes), e.g., $IgG_1$, $IgG_2$, $IgG_3$, $IgG_4$, $IgA_1$, and $IgA_2$. The heavy chain constant domains that correspond to the different classes of immunoglobulins are called $\alpha$, $\delta$, $\varepsilon$, $\gamma$, and $\mu$, respectively. The subunit structures and three-dimensional configurations of different classes of immunoglobulins are well known and described generally in, for example, Abbas et al. Cellular and Mol. Immunology, 4th ed. (W.B. Saunders, Co., 2000). An antibody may be part of a larger fusion molecule, formed by covalent or non-covalent association of the antibody with one or more other proteins or peptides.

The term "IgG subclass modification" as used herein is meant an amino acid modification that converts one amino acid of one IgG isotype to the corresponding amino acid in a different, aligned IgG isotype. For example, because $IgG_1$ comprises a tyrosine and $IgG_2$ a phenylalanine at EU position 296, a F296Y substitution in $IgG_2$ is considered an

IgG subclass modification.

By "non-naturally occurring modification" as used herein is meant an amino acid modification that is not isotypic. For example, because none of the IgGs comprise a glutamic acid at position 332, substitution at position 332 with glutamic acid (332E) in $IgG_1$, $IgG_2$, $IgG_3$, or $IgG_4$ is considered a non-naturally occuring modification.

The terms "full length antibody," "intact antibody" and "whole antibody" are used herein interchangeably to refer to an antibody in its substantially intact form. The terms particularly refer to an antibody with heavy chains that contain an Fc region. The C-terminal lysine (residue 447 according to the EU numbering system) of the antibody my be removed, for example, during purification of the antibody or by recombinant engineering of the nucleic acid encoding the antibody. Antibodies with or without this C-terminal lysine are "full length", "intact" or "whole" as those terms are used herein.

A "naked antibody" for the purposes herein is an antibody that is not conjugated to a cytotoxic moiety or radiolabel.

"Antibody fragments" comprise a portion of an intact antibody, preferably comprising the antigen binding region thereof. In certain embodiments, antibody fragments comprise an Fc region or a portion of Fc region comprising one or more Fc region modification disclosed herein. Examples of antibody fragments include Fab, Fab', $F(ab')_2$, and Fv fragments; diabodies; linear antibodies; single-chain antibody molecules; and multispecific antibodies formed from antibody fragments.

Papain digestion of antibodies produces two identical antigen-binding fragments, called "Fab" fragments, each with a single antigen-binding site, and a residual "Fc" fragment, whose name reflects its ability to crystallize readily. Pepsin treatment yields an $F(ab')_2$ fragment that has two antigen-combining sites and is still capable of cross-linking antigen.

"Fv" is the minimum antibody fragment which contains a complete antigen-binding site. In one embodiment, a two-chain Fv species consists of a dimer of one heavy- and one light-chain variable domain in tight, non-covalent association. In a single-chain Fv (scFv) species, one heavy- and one light-chain variable domain can be covalently linked by a flexible peptide linker such that the light and heavy chains can associate in a "dimeric" structure analogous to that in a two-chain Fv species. It is in this configuration that the three HVRs of each variable domain interact to define an antigen-binding site on the surface of the VH-VL dimer. Collectively, the six HVRs confer antigen-binding specificity to the antibody. However, even a single variable domain (or half of an Fv comprising only three HVRs specific for an antigen) has the ability to recognize and bind antigen, although at a lower affinity than the entire binding site.

The Fab fragment contains the heavy- and light-chain variable domains and also contains the constant domain of the light chain and the first constant domain ($C_H1$) of the heavy chain. Fab' fragments differ from Fab fragments by the addition of a few residues at the carboxy terminus of the heavy chain $C_H1$ domain including one or more cysteines from the antibody hinge region. Fab'-SH is the designation herein for Fab' in which the cysteine residue(s) of the constant domains bear a free thiol group. $F(ab')_2$ antibody fragments originally were produced as pairs of Fab' fragments which have hinge cysteines between them. Other chemical couplings of antibody fragments are also known.

"Single-chain Fv" or "scFv" antibody fragments comprise the $V_H$ and $V_L$ domains of antibody, wherein these domains are present in a single polypeptide chain. Generally, the scFv polypeptide further comprises a polypeptide linker between the $V_H$ and $V_L$ domains which enables the scFv to form the desired structure for antigen binding. For a review of scFv, see, *e.g.,* Pluckthün, in The Pharmacology of Monoclonal Antibodies, vol. 113, Rosenburg and Moore eds., (Springer-Verlag, New York, 1994), pp. 269-315.

The term "diabodies" refers to antibody fragments with two antigen-binding sites, which fragments comprise a heavy-chain variable domain ($V_H$) connected to a light-chain variable domain ($V_L$) in the same polypeptide chain ($V_H$-$V_L$). By using a linker that is too short to allow pairing between the two domains on the same chain, the domains are forced to pair with the complementary domains of another chain and create two antigen-binding sites. Diabodies may be bivalent or bispecific. Diabodies are described more fully in, for example, EP 404,097; WO 1993/01161; Hudson et al., Nat. Med. 9:129-134 (2003); and Hollinger et al., Proc. Natl. Acad. Sci. USA 90: 6444-6448 (1993). Triabodies and tetrabodies are also described in Hudson et al., Nat. Med. 9:129-134 (2003).

The term "monoclonal antibody" as used herein refers to an antibody obtained from a population of substantially homogeneous antibodies, *i.e.,* the individual antibodies comprising the population are identical except for possible mutations, e.g., naturally occurring mutations, that may be present in minor amounts. Thus, the modifier "monoclonal" indicates the character of the antibody as not being a mixture of discrete antibodies. In certain embodiments, such a monoclonal antibody typically includes an antibody comprising a polypeptide sequence that binds a target, wherein the target-binding polypeptide sequence was obtained by a process that includes the selection of a single target binding polypeptide sequence from a plurality of polypeptide sequences. For example, the selection process can be the selection of a unique clone from a plurality of clones, such as a pool of hybridoma clones, phage clones, or recombinant DNA clones. It should be understood that a selected target binding sequence can be further altered, for example, to improve affinity for the target, to humanize the target binding sequence, to improve its production in cell culture, to reduce its immunogenicity *in vivo,* to create a multispecific antibody, etc., and that an antibody comprising the altered target binding sequence is also a monoclonal antibody of this invention. In contrast to polyclonal antibody preparations, which typically include different antibodies directed against different determinants (epitopes), each monoclonal antibody of a monoclonal antibody preparation is directed against a single determinant on an antigen. In addition to their specificity, monoclonal antibody preparations are advantageous in that they are typically uncontaminated by other immunoglobulins.

The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including, for example, the hybridoma method (e.g., Kohler and Milstein, Nature, 256:495-97 (1975); Hongo et al., Hybridoma, 14 (3): 253-260 (1995), Harlow et al., Antibodies: A Laboratory Manual, (Cold Spring Harbor Laboratory Press, 2nd ed. 1988); Hammerling et al., in: Monoclonal Antibodies and T-Cell Hybridomas 563-681 (Elsevier, N.Y., 1981)), recombinant DNA methods (see, e.g., U.S. Patent No. 4,816,567), phage-display technologies (see, e.g., Clackson et al., Nature, 352: 624-628 (1991); Marks et al., J. Mol. Biol. 222: 581-597 (1992); Sidhu et al., J. Mol. Biol. 338(2): 299-310 (2004); Lee et al., J. Mol. Biol. 340(5): 1073-1093 (2004); Fellouse, Proc. Natl. Acad. Sci. USA 101(34): 12467-12472 (2004); and Lee et al., J. Immunol. Methods 284(1-2): 119-132(2004), and technologies for producing human or human-like antibodies in animals that have parts or all of the human immunoglobulin loci or genes encoding human immunoglobulin sequences (see, e.g., WO 1998/24893; WO 1996/34096; WO 1996/33735; WO 1991/10741; Jakobovits et al., Proc. Natl. Acad. Sci. USA 90: 2551 (1993); Jakobovits et al., Nature 362: 255-258 (1993); Bruggemann et al., Year in Immunol. 7:33 (1993); U.S. Patent Nos. 5,545,807; 5,545,806; 5,569,825; 5,625,126; 5,633,425; and 5,661,016; Marks et al., Bio/Technology 10: 779-783 (1992); Lonberg et al., Nature 368: 856-859 (1994); Morrison, Nature 368: 812-813 (1994); Fishwild et al., Nature Biotechnol. 14: 845-851 (1996); Neuberger, Nature Biotechnol. 14: 826 (1996); and Lonberg and Huszar, Intern. Rev. Immunol. 13: 65-93 (1995).

The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (see, e.g.,U.S. Patent No. 4,816,567; and Morrison et al., Proc. Natl. Acad. Sci. USA 81:6851-6855 (1984)). Chimeric antibodies include PRIMATIZED® antibodies wherein the antigen-binding region of the antibody is derived from an antibody produced by, e.g., immunizing macaque monkeys with the antigen of interest.

"Humanized" forms of non-human (*e.g.*, murine) antibodies are chimeric antibodies that contain minimal sequence derived from non-human immunoglobulin. In one embodiment, a humanized antibody is a human immunoglobulin (recipient antibody) in which residues from a HVR of the recipient are replaced by residues from a HVR of a non-human species (donor antibody) such as mouse, rat, rabbit, or nonhuman primate having the desired specificity, affinity, and/or capacity. In some instances, FR residues of the human immunoglobulin are replaced by corresponding non-human residues. Furthermore, humanized antibodies may comprise residues that are not found in the recipient antibody or in the donor antibody. These modifications may be made to further refine antibody performance. In general, a humanized antibody will comprise substantially all of at least one, and typically two, variable domains, in which all or substantially all of the hypervariable loops correspond to those of a non-human immunoglobulin, and all or substantially all of the FRs are those of a human immunoglobulin sequence. The humanized antibody optionally will also comprise at least a portion of an immunoglobulin constant region (Fc), typically that of a human immunoglobulin. For further details, see, *e.g.,* Jones et al., Nature 321:522-525 (1986); Riechmann et al., Nature 332:323-329 (1988); and Presta, Curr. Op. Struct. Biol. 2:593-596 (1992). See also, *e.g.,* Vaswani and Hamilton, Ann. Allergy, Asthma & Immunol. 1:105-115 (1998); Harris, Biochem. Soc. Transactions 23:1035-1038 (1995); Hurle and Gross, Curr. Op. Biotech. 5:428-433 (1994); and U.S. Pat. Nos. 6,982,321 and 7,087,409.

A "human antibody" is one which possesses an amino acid sequence which corresponds to that of an antibody produced by a human and/or has been made using any of the techniques for making human antibodies as disclosed herein. This definition of a human antibody specifically excludes a humanized antibody comprising non-human antigen-binding residues. Human antibodies can be produced using various techniques known in the art, including phage-display libraries. Hoogenboom and Winter, J. Mol. Biol., 227:381 (1991); Marks et al., J. Mol. Biol., 222:581 (1991). Also available for the preparation of human monoclonal antibodies are methods described in Cole et al., Monoclonal Antibodies and Cancer Therapy, Alan R. Liss, p. 77 (1985); Boerner et al., J. Immunol., 147(1):86-95 (1991). See also van Dijk and van de Winkel, Curr. Opin. Pharmacol., 5: 368-74 (2001). Human antibodies can be prepared by administering the antigen to a transgenic animal that has been modified to produce such antibodies in response to antigenic challenge, but whose endogenous loci have been disabled, *e.g.,* immunized xenomice (see, *e.g.,* U.S. Pat. Nos. 6,075,181 and 6,150,584 regarding XENOMOUSE™ technology). See also, for example, Li et al., Proc. Natl. Acad. Sci. USA, 103:3557-3562 (2006) regarding human antibodies generated via a human B-cell hybridoma technology.

The term "hypervariable region," "HVR," or "HV," when used herein refers to the regions of an antibody variable domain which are hypervariable in sequence and/or form structurally defined loops. Generally, antibodies comprise six HVRs; three in the $V_H$ (H1, H2, H3), and three in the $V_L$ (L1, L2, L3). In native antibodies, H3 and L3 display the most diversity of the six HVRs, and H3 in particular is believed to play a unique role in conferring fine specificity to antibodies.

See, *e.g.,* Xu et al., Immunity 13:37-45 (2000); Johnson and Wu, in Methods in Molecular Biology 248:1-25 (Lo, ed., Human Press, Totowa, NJ, 2003). Indeed, naturally occurring camelid antibodies consisting of a heavy chain only are functional and stable in the absence of light chain. See, *e.g.,* Hamers-Casterman et al., Nature 363:446-448 (1993); Sheriff et al., Nature Struct. Biol. 3:733-736 (1996).

A number of HVR delineations are in use and are encompassed herein. The Kabat Complementarity Determining Regions (CDRs) are based on sequence variability and are the most commonly used (Kabat et al., Sequences of Proteins of Immunological Interest, 5th Ed. Public Health Service, National Institutes of Health, Bethesda, MD. (1991)). Chothia refers instead to the location of the structural loops (Chothia and Lesk J. Mol. Biol. 196:901-917 (1987)). The AbM HVRs represent a compromise between the Kabat HVRs and Chothia structural loops, and are used by Oxford Molecular's AbM antibody modeling software. The "contact" HVRs are based on an analysis of the available complex crystal structures. The residues from each of these HVRs are noted below.

| Loop | | Kabat | | AbM | | Chothia | | Contact |
|---|---|---|---|---|---|---|---|---|
| L1 | | L24-L34 | | L24-L34 | | L26-L32 | | L30-L36 |
| L2 | | L50-L56 | | L50-L56 | | L50-L52 | | L46-L55 |
| L3 | | L89-L97 | | L89-L97 | | L91-L96 | | L89-L96 |
| H1† | | H31-H35B | | H26-H35B | | H26-H32 | | H30-H35B |
| H1‡ | | H31-H35 | | H26-H35 | | H26-H32 | | H30-H35 |
| H2 | | H50-H65 | | H50-H58 | | H53-H55 | | H47-H58 |
| H3 | | H95-H102 | | H95-H102 | | H96-H101 | | H93-H101 |
| †Kabat Numbering<br>‡Chothia Numbering | | | | | | | | |

HVRs may comprise "extended HVRs" as follows: 24-36 or 24-34 (L1), 46-56 or 50-56 (L2) and 89-97 or 89-96 (L3) in the VL and 26-35 (H1), 50-65 or 49-65 (H2) and 93-102, 94-102, or 95-102 (H3) in the VH. The variable domain residues are numbered according to Kabat et al., supra, for each of these definitions.

"Framework" or "FR" residues are those variable domain residues other than the HVR residues as herein defined.

The term "variable domain residue numbering as in Kabat" or "amino acid position numbering as in Kabat," and variations thereof, refers to the numbering system used for heavy chain variable domains or light chain variable domains of the compilation of antibodies in Kabat et al., *supra.* Using this numbering system, the actual linear amino acid sequence may contain fewer or additional amino acids corresponding to a shortening of, or insertion into, a FR or HVR of the variable domain. For example, a heavy chain variable domain may include a single amino acid insert (residue 52a according to Kabat) after residue 52 of H2 and inserted residues (e.g. residues 82a, 82b, and 82c, etc. according to Kabat) after heavy chain FR residue 82. The Kabat numbering of residues may be determined for a given antibody by alignment at regions of homology of the sequence of the antibody with a "standard" Kabat numbered sequence.

The Kabat numbering system is generally used when referring to a residue in the variable domain (approximately residues 1-107 of the light chain and residues 1-113 of the heavy chain) (*e.g,* Kabat et al., Sequences of Immunological Interest. 5th Ed. Public Health Service, National Institutes of Health, Bethesda, Md. (1991)). The "EU numbering system" or "EU index" is generally used when referring to a residue in an immunoglobulin heavy chain constant region (*e.g.,* the EU index reported in Kabat *et al., supra).* The "EU index as in Kabat" refers to the residue numbering of the human $IgG_1$ EU antibody. Unless stated otherwise herein, references to residue numbers in the variable domain of antibodies means residue numbering by the Kabat numbering system. Unless stated otherwise herein, references to residue numbers in the constant domain of antibodies means residue numbering by the EU numbering system (*see e.g.,* PCT Publication No. WO2006073941).

An "affinity matured" antibody is one with one or more alterations in one or more HVRs thereof which result in an improvement in the affinity of the antibody for antigen, compared to a parent antibody which does not possess those alteration(s). In one embodiment, an affinity matured antibody has nanomolar or even picomolar affinities for the target antigen. Affinity matured antibodies may be produced using certain procedures known in the art. For example, Marks et al. Bio/Technology 10:779-783 (1992) describes affinity maturation by $V_H$ and $V_L$ domain shuffling. Random mutagenesis of HVR and/or framework residues is described by, for example, Barbas et al. Proc Nat. Acad. Sci. USA 91:3809-3813 (1994); Schier et al. Gene 169:147-155 (1995); Yelton et al. J. Immunol. 155:1994-2004 (1995); Jackson et al., J. Immunol. 154(7):3310-9 (1995); and Hawkins et al, J. Mol. Biol. 226:889-896 (1992).

Antibody "effector functions" refer to those biological activities attributable to the Fc region (a native sequence Fc region or amino acid sequence variant Fc region) of an antibody, and vary with the antibody isotype. Examples of antibody effector functions include: C1q binding and complement dependent cytotoxicity (CDC); Fc receptor binding; antibody-dependent cell-mediated cytotoxicity (ADCC); phagocytosis; down regulation of cell surface receptors (*e.g.* B cell receptor); and B cell activation.

The term "Fc region" herein is used to define a C-terminal region of an immunoglobulin heavy chain, including native sequence Fc regions and variant Fc regions. Although the boundaries of the Fc region of an immunoglobulin heavy chain might vary, the human IgG heavy chain Fc region is usually defined to stretch from an amino acid residue at position Cys226, or from Pro230, to the carboxyl-terminus thereof. The C-terminal lysine (residue 447 according to the EU numbering system) of the Fc region may be removed, for example, during production or purification of the antibody, or by recombinantly engineering the nucleic acid encoding a heavy chain of the antibody. Accordingly, a composition of intact antibodies may comprise antibody populations with all K447 residues removed, antibody populations with no K447 residues removed, and antibody populations having a mixture of antibodies with and without the K447 residue. In certain embodiments, the Fc region of an immunoglobulin comprises two constant domains, $C_H2$ and $C_H3$.

The "$V_H$ domain" of a human IgG usually extends from about amino acid 1 to about amino acid 113.

The "$C_H2$ domain" of a human IgG Fc region (also referred to as "Cy2" domain) usually extends from about amino acid 231 to about amino acid 340. The $C_H2$ domain is unique in that it is not closely paired with another domain. Rather, two N-linked branched carbohydrate chains are interposed between the two $C_H2$ domains of an intact native IgG molecule. It has been speculated that the carbohydrate may provide a substitute for the domain-domain pairing and help stabilize the $C_H2$ domain. Burton, Molec. Immunol. 22:161-206 (1985).

The "$C_H3$ domain" comprises the stretch of residues C-terminal to a $C_H2$ domain in an Fc region (i.e. from about amino acid residue 341 to about amino acid residue 447 of an IgG).

A "functional Fc region" possesses an "effector function" of a native sequence Fc region. Exemplary "effector functions" include Fc receptor binding; C1q binding; CDC; ADCC; phagocytosis; down regulation of cell surface receptors (*e.g.* B cell receptor; BCR), *etc.* Such effector functions generally require the Fc region to be combined with a binding domain (*e.g.*, an antibody variable domain) and can be assessed using various assays.

A "variant Fc region" comprises an amino acid sequence which differs from that of a native sequence Fc region by virtue of at least one amino acid modification,

"Fc receptor" or "FcR" describes a receptor that binds to the Fc region of an antibody. In some embodiments, an FcR is a native human FcR. In some embodiments, an FcR is one which binds an IgG antibody (a gamma receptor) and includes receptors of the FcγRI, FcγRII, and FcγRIII subclasses, including allelic variants and alternatively spliced forms of those receptors. FcγRII receptors include FcγRIIA (an "activating receptor") and FcγRIIB (an "inhibiting receptor"), which have similar amino acid sequences that differ primarily in the cytoplasmic domains thereof. Activating receptor FcγRIIA contains an immunoreceptor tyrosine-based activation motif (ITAM) in its cytoplasmic domain. Inhibiting receptor FcyRIIB contains an immunoreceptor tyrosine-based inhibition motif (ITIM) in its cytoplasmic domain. (see, *e.g.,* Daëron, Annu. Rev. Immunol. 15:203-234 (1997)). FcRs are reviewed, for example, in Ravetch and Kinet, Annu. Rev. Immunol 9:457-92 (1991); Capel et al., Immunomethods 4:25-34 (1994); and de Haas et al., J. Lab. Clin. Med. 126:330-41 (1995). Other FcRs, including those to be identified in the future, are encompassed by the term "FcR" herein.

"Complement dependent cytotoxicity" or "CDC" refers to the lysis of a target cell in the presence of complement. Activation of the classical complement pathway is initiated by the binding of the first component of the complement system (C1q) to antibodies (of the appropriate subclass), which are bound to their cognate antigen. To assess complement activation, a CDC assay, *e.g.,* as described in Gazzano-Santoro et al., J. Immunol. Methods 202:163 (1996), may be performed. Polypeptide variants with altered Fc region amino acid sequences (polypeptides with a variant Fc region) and increased or decreased C1q binding capability are described, *e.g.,* in US Patent No. 6,194,551 B1 and WO 1999/51642. See also, *e.g.,* Idusogie et al. J. Immunol. 164: 4178-4184 (2000).

"Binding affinity" generally refers to the strength of the sum total of noncovalent interactions between a single binding site of a molecule (*e.g.,* an antibody) and its binding partner (*e.g.,* an antigen). Unless indicated otherwise, as used herein, "binding affinity" refers to intrinsic binding affinity which reflects a 1:1 interaction between members of a binding pair (*e.g.*, antibody and antigen). The affinity of a molecule X for its partner Y can generally be represented by the dissociation constant (Kd), the reciprocal of the association constant (Ka). Affinity can be measured by common methods known in the art, including those described herein. Low-affinity antibodies generally bind antigen slowly and/or tend to dissociate readily, whereas high-affinity antibodies generally bind antigen faster and/or tend to remain bound longer. A variety of methods of measuring binding affinity are known in the art, any of which can be used for purposes of the present invention. Specific illustrative and exemplary embodiments for measuring binding affinity are described in the following.

In certain embodiments, the "$K_D$," "$K_d$," "Kd" or "Kd value" according to this invention is measured by using surface plasmon resonance assays using a BIACORE®-2000 or a BIACORE®-3000 (BIAcore, Inc., Piscataway, NJ) at 25°C with immobilized antigen CM5 chips at -10 response units (RU). Briefly, carboxymethylated dextran biosensor chips (CM5, BIACORE, Inc.) are activated with *N*-ethyl-*N*'- (3-dimethylaminopropyl)-carbodiimide hydrochloride (EDC) and N-hydroxysuccinimide (NHS) according to the supplier's instructions. Antigen is diluted with 10 mM sodium acetate, pH 4.8, to 5 μg/ml (-0.2 μM) before injection at a flow rate of 5 μl/minute to achieve approximately 10 response units (RU) of coupled protein. Following the injection of antigen, 1 M ethanolamine is injected to block unreacted groups. For kinetics measurements, serial dilutions of polypeptide, *e.g.,* full length antibody, are injected in PBS with 0.05% TWEEN-20™ surfactant (PBST) at 25°C at a flow rate of approximately 25 μl/min. Association rates ($k_{on}$) and dissociation rates ($k_{off}$) are calculated using a simple one-to-one Langmuir binding model (BIACORE® Evaluation Software version 3.2) by simultaneously fitting the association and dissociation sensorgrams. The equilibrium dissociation constant (Kd) is calculated as the ratio $k_{off}/k_{on}$. *See, e.g.,* Chen et al., J. Mol. Biol. 293:865-881 (1999). If the on-rate exceeds $10^6$ $M^{-1}$ $s^{-1}$ by the surface plasmon resonance assay above, then the on-rate can be determined by using a fluorescent quenching technique that measures the increase or decrease in fluorescence emission intensity (excitation = 295 nm; emission = 340 nm, 16 nm band-pass) at 25°C of a 20 nM anti-antigen antibody in PBS, pH 7.2, in the presence of increasing concentrations of antigen as measured in a spectrometer, such as a stop-flow equipped spectrophometer (Aviv Instruments) or a 8000-series SLM-AMINCO ™ spectrophotometer (ThermoSpectronic) with a stirred cuvette.

An "on-rate," "rate of association," "association rate," or "$k_{on}$" according to this invention can also be determined as described above using a BIACORE®-2000 or a BIACORE®-3000 system (BIAcore, Inc., Piscataway, NJ).

The term "substantially similar" or "substantially the same," as used herein, denotes a sufficiently high degree of similarity between two numeric values such that one of skill in the art would consider the difference between the two values to be of little or no biological and/or statistical significance within the context of the biological characteristic measured by said values (*e.g.,* Kd values). In certain embodiments, the difference between said two values is, for example, less than about 50%, less than about 40%, less than about 30%, less than about 20%, and/or less than about 10% as a function of the reference/comparator value.

The phrase "substantially reduced," or "substantially different," as used herein, denotes a sufficiently high degree of difference between two numeric values such that one of skill in the art would consider the difference between the two values to be of statistical significance within the context of the biological characteristic measured by said values (*e.g.,* Kd values). In certain embodiments, the difference between said two values is, for example, greater than about 10%, greater than about 20%, greater than about 30%, greater than about 40%, and/or greater than about 50% as a function of the value for the reference/comparator molecule.

"Purified" means that a molecule is present in a sample at a concentration of at least 95% by weight, or at least 98% by weight of the sample in which it is contained.

An "isolated" nucleic acid molecule is a nucleic acid molecule that is separated from at least one other nucleic acid molecule with which it is ordinarily associated, for example, in its natural environment. An isolated nucleic acid molecule further includes a nucleic acid molecule contained in cells that ordinarily express the nucleic acid molecule, but the nucleic acid molecule is present extrachromosomally or at a chromosomal location that is different from its natural chromosomal location.

The term "vector," as used herein, is intended to refer to a nucleic acid molecule capable of transporting another nucleic acid to which it has been linked. One type of vector is a "plasmid," which refers to a circular double stranded DNA into which additional DNA segments may be ligated. Another type of vector is a phage vector. Another type of vector is a viral vector, wherein additional DNA segments may be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) can be integrated into the genome of a host cell upon introduction into the host cell, and thereby are replicated along with the host genome. Moreover, certain vectors are capable of directing the expression of genes to which they are operatively linked. Such vectors are referred to herein as "recombinant expression vectors," or simply, "expression vectors." In general, expression vectors of utility in recombinant DNA techniques are often in the form of plasmids. In the present specification, "plasmid" and "vector" may be used interchangeably as the plasmid is the most commonly used form of vector.

"Polynucleotide," or "nucleic acid," as used herein, refer to polymers of nucleotides of any length, and include DNA and RNA. The nucleotides can be deoxyribonucleotides, ribonucleotides, modified nucleotides or bases, and/or their analogs, or any substrate that can be incorporated into a polymer by DNA or RNA polymerase or by a synthetic reaction. A polynucleotide may comprise modified nucleotides, such as methylated nucleotides and their analogs. If present, modification to the nucleotide structure may be imparted before or after assembly of the polymer. The sequence of nucleotides may be interrupted by non-nucleotide components. A polynucleotide may comprise modification(s) made after synthesis, such as conjugation to a label. Other types of modifications include, for example, "caps," substitution of one or more of the naturally occurring nucleotides with an analog, internucleotide modifications such as, for example, those with uncharged linkages (*e.g*., methyl phosphonates, phosphotriesters, phosphoamidates, carbamates, *etc*.) and with charged linkages (*e.g.,* phosphorothioates, phosphorodithioates, *etc*.), those containing pendant moieties, such as, for example, proteins (*e.g*., nucleases, toxins, antibodies, signal peptides, poly-L-lysine, *etc*.), those with intercalators (*e.g.,* acridine, psoralen, *etc*.), those containing chelators (*e.g.,* metals, radioactive metals, boron, oxidative metals, *etc*.),

those containing alkylators, those with modified linkages (*e.g.,* alpha anomeric nucleic acids, *etc.*), as well as unmodified forms of the polynucleotides(s). Further, any of the hydroxyl groups ordinarily present in the sugars may be replaced, for example, by phosphonate groups, phosphate groups, protected by standard protecting groups, or activated to prepare additional linkages to additional nucleotides, or may be conjugated to solid or semi-solid supports. The 5' and 3' terminal OH can be phosphorylated or substituted with amines or organic capping group moieties of from 1 to 20 carbon atoms. Other hydroxyls may also be derivatized to standard protecting groups. Polynucleotides can also contain analogous forms of ribose or deoxyribose sugars that are generally known in the art, including, for example, 2'-O-methyl-, 2'-O-allyl-, 2'-fluoro- or 2'-azido-ribose, carbocyclic sugar analogs, $\alpha$-anomeric sugars, epimeric sugars such as arabinose, xyloses or lyxoses, pyranose sugars, furanose sugars, sedoheptuloses, acyclic analogs, and basic nucleoside analogs such as methyl riboside. One or more phosphodiester linkages may be replaced by alternative linking groups. These alternative linking groups include, but are not limited to, embodiments wherein phosphate is replaced by P(O)S ("thioate"), P(S)S ("dithioate"), (O)$NR_2$ ("amidate"), P(O)R, P(O)OR', CO, or CH2 ("formacetal"), in which each R or R' is independently H or substituted or unsubstituted alkyl (1-20 C) optionally containing an ether (-O-) linkage, aryl, alkenyl, cycloalkyl, cycloalkenyl or araldyl. Not all linkages in a polynucleotide need be identical. The preceding description applies to all polynucleotides referred to herein, including RNA and DNA.

"Oligonucleotide," as used herein, generally refers to short, generally single-stranded, generally synthetic polynucleotides that are generally, but not necessarily, less than about 200 nucleotides in length. The terms "oligonucleotide" and "polynucleotide" are not mutually exclusive. The description above for polynucleotides is equally and fully applicable to oligonucleotides.

The expression "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

As used herein, the expressions "cell," "cell line," and "cell culture" are used interchangeably and all such designations include progeny. Thus, the words "transformants" and "transformed cells" include the primary subject cell and cultures derived therefrom without regard for the number of transfers. It is also understood that all progeny may not be precisely identical in DNA content, due to deliberate or inadvertent mutations. Mutant progeny that have the same function or biological activity as screened for in the originally transformed cell are included. Where distinct designations are intended, it will be clear from the context.

As used herein, "codon set" refers to a set of different nucleotide triplet sequences used to encode desired variant amino acids. A set of oligonucleotides can be synthesized, for example, by solid phase synthesis, including sequences that represent all possible combinations of nucleotide triplets provided by the codon set and that will encode the desired group of amino acids. A standard form of codon designation is that of the IUB code, which is known in the art and described herein. A codon set typically is represented by 3 capital letters in italics, eg. NNK, NNS, XYZ, DVK and the like. A "non-random codon set", as used herein, thus refers to a codon set that encodes select amino acids that fulfill partially, preferably completely, the criteria for amino acid selection as described herein. Synthesis of oligonucleotides with selected nucleotide "degeneracy" at certain positions is well known in that art, for example the TRIM approach (Knappek et al. (1999) J. Mol. Biol. 296:57-86); Garrard & Henner (1993) Gene 128:103). Such sets of oligonucleotides having certain codon sets can be synthesized using commercial nucleic acid synthesizers (available from, for example, Applied Biosystems, Foster City, CA), or can be obtained commercially (for example, from Life Technologies, Rockville, MD). Therefore, a set of oligonucleotides synthesized having a particular codon set will typically include a plurality of oligonucleotides with different sequences, the differences established by the codon set within the overall sequence. Oligonucleotides, as used according to the invention, have sequences that allow for hybridization to a variable domain nucleic acid template and also can, but does not necessarily, include restriction enzyme sites useful for, for example, cloning purposes.

The expression "linear antibodies" refers to the antibodies described in Zapata et al. (1995 Protein Eng, 8(10): 1057-1062). Briefly, these antibodies comprise a pair of tandem Fd segments (VH-CH1-VH-CH1) which, together with complementary light chain polypeptides, form a pair of antigen binding regions. Linear antibodies can be bispecific or monospecific.

As used herein, "library" refers to a plurality of antibody or antibody fragment sequences (for example, variant IgGs of the invention), or the nucleic acids that encode these sequences, the sequences being different in the combination of variant amino acids that are introduced into these sequences according to the methods of the invention.

"Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full length of the sequences being compared. For purposes herein, however, % amino acid sequence identity values are generated using the sequence comparison computer program ALIGN-2. The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, California, or may be compiled from the source code. The ALIGN-2 program should be compiled for use on a UNIX operating system, preferably digital UNIX V4.0D. All sequence comparison parameters are set by the ALIGN-2 program and do not vary.

In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

100 times the fraction X/Y

where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

The term "pharmaceutical composition" refers to a preparation which is in such form as to permit the biological activity of the active ingredient to be effective, and which contains no additional components which are unacceptably toxic to a subject to which the formulation would be administered. Such formulations may be sterile.

A "sterile" formulation is aseptic or free from all living microorganisms and their spores.

"Carriers" as used herein include pharmaceutically acceptable carriers, excipients, or stabilizers which are nontoxic to the cell or mammal being exposed thereto at the dosages and concentrations employed. Often the physiologically acceptable carrier is an aqueous pH buffered solution. Examples of physiologically acceptable carriers include buffers such as phosphate, citrate, and other organic acids; antioxidants including ascorbic acid; low molecular weight (less than about 10 residues) polypeptide; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN™, polyethylene glycol (PEG), and PLURONICS™.

## Antibodies

The present application relates to variant IgG immunoglobulins that include amino acid modifications that alter the biological properties of the IgG. The variant immunoglobulins of the present application include antibodies that display altered binding to protein A compared to the wild-type antibodies.

Antibodies are proteins which exhibit binding specificity to a specific antigen. Native antibodies are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies between the heavy chains of different immunoglobulin isotypes. Each heavy and light chain also has regularly spaced intrachain disulfide bridges. Each heavy chain has at one end a variable domain ($V_H$) followed by a number of constant domains. Each light chain has a variable domain at one end ($V_L$) and a constant domain at its other end; the constant domain of the light chain is aligned with the first constant domain of the heavy chain, and the light chain variable domain is aligned with the variable domain of the heavy chain. Particular amino acid residues are believed to form an interface between the light and heavy chain variable domains.

Depending on the amino acid sequence of the constant region of their heavy chains, antibodies or immunoglobulins can be assigned to different classes. There are five major classes of immunoglobulins: IgA, IgD, IgE, IgG and IgM, and several of these may be further divided into subclasses (isotypes), *e.g.* $IgG_1$, $IgG_2$, $IgG_3$, and $IgG_4$; $IgA_1$ and $IgA_2$. A variety of human $IgG_1$, $IgG_2$, $IgG_3$, and $IgG_4$ allotypes have been described (reviewed by M.-P. LeFranc and G. LeFranc in: "The Human IgG Subclasses," F. Shakib (ed.), pp. 43-78, Pergamon Press, Oxford (1990)). The different isotypes of the IgG class, including $IgG_1$, $IgG_{2s}$, $IgG_3$, and $IgG_4$, have unique physical, biological, and clinical properties. Human $IgG_1$ is the most commonly used antibody for therapeutic purposes, and the majority of engineering studies have been constructed in this context.

### *Antibody Fragments*

The present invention encompasses antibody fragments. Of particular interest are antibodies that comprise the variable regions of the heavy and light chains. In certain embodiments, the antibody fragments are the fragments of variant immunoglobulins (IgGs) comprising Fc regions. Antibody fragments may be generated by traditional means, such as enzymatic digestion, or by recombinant techniques.

Various techniques have been developed for the production of antibody fragments. Traditionally, these fragments were derived via proteolytic digestion of intact antibodies *(see, e.g.,* Morimoto et al., Journal of Biochemical and Biophysical Methods 24:107-117 (1992); and Brennan et al., Science, 229:81 (1985)). However, these fragments can now be produced directly by recombinant host cells. Fab, Fv and ScFv antibody fragments can all be expressed in and secreted from *E. coli,* thus allowing the facile production of large amounts of these fragments. Antibody fragments can be isolated from the antibody phage libraries. In certain embodiments. Fab'-SH fragments can be directly recovered from *E. coli* and chemically coupled to form $F(ab')_2$ fragments (Carter et al., Bio/Technology 10:163-167 (1992)). According to another approach, $F(ab')_2$ fragments can be isolated directly from recombinant host cell culture. Other techniques for the production of antibody fragments will be apparent to the skilled practitioner. The antibody fragment may also be a "linear antibody", *e.g*., as described in U.S. Pat. No. 5,641,870, for example. Such linear antibodies may be monospecific or bispecific.

### *Humanized Antibodies*

The invention encompasses humanized antibodies. In certain embodiments, the humanized antibodies are humanized variant IgGs with one or more amino acid modifications in the Fc region relative to wild-type IgG. Various methods for humanizing non-human antibodies are known in the art. For example, a humanized antibody can have one or more amino acid residues introduced into it from a source which is non-human. These non-human amino acid residues are often referred to as "import" residues, which are typically taken from an "import" variable domain. Humanization can be essentially performed following the method of Winter and co-workers (Jones et al. (1986) Nature 321:522-525; Riechmann et al. (1988) Nature 332:323-327; Verhoeyen et al. (1988) Science 239:1534-1536), by substituting hypervariable region sequences for the corresponding sequences of a human antibody. Accordingly, such "humanized" antibodies are chimeric antibodies (U.S. Patent No. 4,816,567) wherein substantially less than an intact human variable domain has been substituted by the corresponding sequence from a non-human species. In practice, humanized antibodies are typically human antibodies in which some hypervariable region residues and possibly some FR residues are substituted by residues from analogous sites in rodent antibodies.

The choice of human variable domains, both light and heavy, to be used in making the humanized antibodies can be important to reduce antigenicity. According to the so-called "best-fit" method, the sequence of the variable domain of a rodent antibody is screened against the entire library of known human variable-domain sequences. The human sequence which is closest to that of the rodent is then accepted as the human framework for the humanized antibody. See, e.g., Sims et al. (1993) J. Immunol. 151:2296; Chothia et al. (1987) J. Mol. Biol. 196:901. Another method uses a particular framework derived from the consensus sequence of all human antibodies of a particular subgroup of light or heavy chains. The same framework may be used for several different humanized antibodies. See, e.g., Carter et al. (1992) Proc. Natl. Acad. Sci. USA, 89:4285; Presta et al. (1993) J. Immunol., 151:2623.

It is further generally desirable that antibodies be humanized with retention of high affinity for the antigen and other favorable biological properties. To achieve this goal, according to one method, humanized antibodies are prepared by a process of analysis of the parental sequences and various conceptual humanized products using three-dimensional models of the parental and humanized sequences. Three-dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, *i.e.,* the analysis of residues that influence the ability of the candidate immunoglobulin to bind its antigen. In this way, FR residues can be selected and combined from the recipient and import sequences so that the desired antibody characteristic, such as increased affinity for the target antigen(s), is achieved. In general, the hypervariable region residues are directly and most substantially involved in influencing antigen binding.

### *Human Antibodies*

In certain embodiments, the human antibodies of the present invention are human variant IgGs with one or more amino acid modifications in the $V_H$ region relative to wild-type IgG. Human antibodies can be constructed by combining Fv clone variable domain sequence(s) selected from human-derived phage display libraries with known human constant domain sequences(s) as described above. Alternatively, human monoclonal antibodies can be made by the hybridoma method. Human myeloma and mouse-human heteromyeloma cell lines for the production of human monoclonal antibodies have been described, for example, by Kozbor J. Immunol., 133: 3001 (1984); Brodeur et al., Monoclonal Antibody Production Techniques and Applications, pp. 51-63 (Marcel Dekker, Inc., New York, 1987); and Boerner et al., J. Immunol., 147: 86 (1991).

It is now possible to produce transgenic animals (e.g. mice) that are capable, upon immunization, of producing a full repertoire of human antibodies in the absence of endogenous immunoglobulin production. For example, it has been described that the homozygous deletion of the antibody heavy-chain joining region (JH) gene in chimeric and germ-line mutant mice results in complete inhibition of endogenous antibody production. Transfer of the human germ-line immunoglobulin gene array in such germ-line mutant mice will result in the production of human antibodies upon antigen challenge. *See, e.g.,* Jakobovits et al., Proc. Natl. Acad. Sci USA, 90: 2551 (1993); Jakobovits et al., Nature, 362: 255 (1993); Bruggermann et al., Year in Immunol., 7: 33 (1993).

Gene shuffling can also be used to derive human antibodies from non-human, e.g. rodent, antibodies, where the human antibody has similar affinities and specificities to the starting non-human antibody. According to this method, which is also called "epitope imprinting", either the heavy or light chain variable region of a non-human antibody fragment obtained by phage display techniques as described herein is replaced with a repertoire of human V domain genes, creating a population of non-human chain/human chain scFv or Fab chimeras. Selection with antigen results in isolation of a non-human chain/human chain chimeric scFv or Fab wherein the human chain restores the antigen binding site destroyed upon removal of the corresponding non-human chain in the primary phage display clone, i.e. the epitope governs (imprints) the choice of the human chain partner. When the process is repeated in order to replace the remaining non-human chain, a human antibody is obtained (see PCT WO 93/06213 published April 1, 1993). Unlike traditional humanization of non-human antibodies by CDR grafting, this technique provides completely human antibodies, which have no FR or CDR residues of non-human origin.

### *Bispecific Antibodies*

Bispecific antibodies are monoclonal antibodies that have binding specificities for at least two different antigens. In certain embodiments, the bispecific antibodies are bispecific antibodies with one or more amino acid modifications in the $V_H$ region relative to wild-type antibody. In certain embodiments, bispecific antibodies are human or humanized antibodies. Bispecific antibodies may also be used to localize cytotoxic agents to cells which express a target antigen. These antibodies possess a target-antigen-binding arm and an arm which binds a cytotoxic agent, such as, *e.g.*, saporin, anti-interferon-a, vinca alkaloid, ricin A chain, methotrexate or radioactive isotope hapten. In certain antibodies, the binding specificities are for IL-4 and IL-13. Bispecific antibodies can be prepared as full length antibodies or antibody fragments.

Methods for making bispecific antibodies are known in the art. Traditionally, the recombinant production of bispecific antibodies is based on the co-expression of two immunoglobulin heavy chain-light chain pairs, where the two heavy chains have different specificities (Milstein and Cuello, Nature, 305: 537 (1983)). Because of the random assortment of immunoglobulin heavy and light chains, these hybridomas (quadromas) produce a potential mixture of 10 different antibody molecules, of which only one has the correct bispecific structure. The purification of the correct molecule, which is usually done by affinity chromatography steps, is rather cumbersome, and the product yields are low. Similar procedures are disclosed in WO 93/08829 published May 13, 1993, and in Traunecker et al., EMBO J., 10: 3655 (1991).

According to a different approach, antibody variable domains with the desired binding specificities (antibody-antigen combining sites) are fused to immunoglobulin constant domain sequences. The fusion, for example, is with an immunoglobulin heavy chain constant domain, comprising at least part of the hinge, $C_H2$, and $C_H3$ regions. In certain embodiments, the first heavy-chain constant region ($C_H1$), containing the site necessary for light chain binding, is present in at least one of the fusions. DNAs encoding the immunoglobulin heavy chain fusions and, if desired, the immunoglobulin light chain, are inserted into separate expression vectors, and are co-transfected into a suitable host organism. This provides for great flexibility in adjusting the mutual proportions of the three polypeptide fragments in embodiments when unequal ratios of the three polypeptide chains used in the construction provide the optimum yields. It is, however, possible to insert the coding sequences for two or all three polypeptide chains in one expression vector when the expression of at least two polypeptide chains in equal ratios results in high yields or when the ratios are of no particular significance.

In one embodiment of this approach, the bispecific antibodies are composed of a hybrid immunoglobulin heavy chain with a first binding specificity in one arm, and a hybrid immunoglobulin heavy chain-light chain pair (providing a second binding specificity) in the other arm. It was found that this asymmetric structure facilitates the separation of the desired bispecific compound from unwanted immunoglobulin chain combinations, as the presence of an immunoglobulin light chain in only one half of the bispecific molecule provides for a facile way of separation. This approach is disclosed in WO 94/04690. For further details of generating bispecific antibodies see, for example, Suresh et al., Methods in Enzymology, 121:210 (1986).

According to another approach, the interface between a pair of antibody molecules can be engineered to maximize the percentage of heterodimers which are recovered from recombinant cell culture. The interface comprises at least a part of the $C_H3$ domain of an antibody constant domain. In this method, one or more small amino acid side chains from the interface of the first antibody molecule are replaced with larger side chains (e.g. tyrosine or tryptophan). Compensatory "cavities" of identical or similar size to the large side chain(s) are created on the interface of the second antibody molecule by replacing large amino acid side chains with smaller ones (*e.g.* alanine or threonine). This provides a mechanism for increasing the yield of the heterodimer over other unwanted end-products such as homodimers.

Bispecific antibodies include cross-linked or "heteroconjugate" antibodies. For example, one of the antibodies in the heteroconjugate can be coupled to avidin, the other to biotin. Such antibodies have, for example, been proposed to target immune system cells to unwanted cells (US Patent No. 4,676,980), and for treatment of HIV infection (WO 91/00360, WO 92/00373, and EP 03089). Heteroconjugate antibodies may be made using any convenient cross-linking method. Suitable cross-linking agents are well known in the art, and are disclosed in US Patent No. 4,676,980, along with a number of cross-linking techniques.

The "diabody" technology described by Hollinger et al., Proc. Natl. Acad. Sci. USA, 90:6444-6448 (1993) has provided an alternative mechanism for making bispecific antibody fragments. The fragments comprise a heavy-chain variable domain ($V_H$) connected to a light-chain variable domain ($V_L$) by a linker which is too short to allow pairing between the two domains on the same chain. Accordingly, the $V_H$ and $V_L$ domains of one fragment are forced to pair with the complementary $V_L$ and $V_H$ domains of another fragment, thereby forming two antigen-binding sites. Another strategy for making bispecific antibody fragments by the use of single-chain Fv (sFv) dimers has also been reported. See Gruber et al., J. Immunol., 152:5368 (1994).

Antibodies with more than two valencies are contemplated. For example, trispecific antibodies can be prepared. Tutt et al. J. Immunol. 147: 60 (1991).

### *Multivalent Antibodies*

A multivalent antibody may be internalized (and/or catabolized) faster than a bivalent antibody by a cell expressing an antigen to which the antibodies bind. The antibodies of the present invention can be multivalent antibodies (which are other than of the IgM class) with three or more antigen binding sites (e.g. tetravalent antibodies), which can be readily produced by recombinant expression of nucleic acid encoding the polypeptide chains of the antibody. The multivalent antibody can comprise a dimerization domain and three or more antigen binding sites. In certain embodiments, the dimerization domain comprises (or consists of) an Fc region or a hinge region. In this scenario, the antibody will comprise an Fc region and three or more antigen binding sites amino-terminal to the Fc region. In certain embodiments, a multivalent antibody comprises (or consists of) three to about eight antigen binding sites. In one such embodiment, a multivalent antibody comprises (or consists of) four antigen binding sites. The multivalent antibody comprises at least one polypeptide chain (for example, two polypeptide chains), wherein the polypeptide chain(s) comprise two or more variable domains. For instance, the polypeptide chain(s) may comprise $VD_1$-$(X_1)_n$-$VD_2$-$(X_2)_n$-Fc, wherein $VD_1$ is a first variable domain, $VD_2$ is a second variable domain, Fc is one polypeptide chain of an Fc region, $X_1$ and $X_2$ represent an amino acid or polypeptide, and n is 0 or 1. For instance, the polypeptide chain(s) may comprise: $V_H$-$C_H1$-flexible linker-$V_H$-$C_H1$-Fc region chain; or $V_H$-$C_H1$-$V_H$-$C_H1$-Fc region chain. The multivalent antibody herein may further comprise at least two (for example, four) light chain variable domain polypeptides. The multivalent antibody herein may, for instance, comprise from about two to about eight light chain variable domain polypeptides. The light chain variable domain polypeptides contemplated here comprise a light chain variable domain and, optionally, further comprise a $C_L$ domain.

### *Single-Domain Antibodies*

In some embodiments, an antibody of the invention is a single-domain antibody comprising Fc region. In certain embodiments, the single-domain antibody has one or more amino acid modifications in the Fc region relative to wild-type IgG. A single-domain antibody is a single polyeptide chain comprising all or a portion of the heavy chain variable domain or all or a portion of the light chain variable domain of an antibody.

***Antibody Modifications***

In certain embodiments, amino acid sequence modification(s) of the immunoglobulins described herein are contemplated. In certain embodiments, modifications comprise one or more amino acid modifications to the variant IgGs of the present invention. In certain embodiments, it may be desirable to further alter the binding affinity, *in vivo* half-life and/or other biological properties of the variant IgGs of the present invention. In certain embodiments, amino acid modifications comprise one or more amino acid modifications in the Fc region not described herein. Modified amino acid sequences of the variant IgGs may be prepared by introducing appropriate changes into the nucleotide sequence encoding the antibody, or by peptide synthesis. Such modifications include, for example, deletions from, and/or insertions into and/or substitutions of, residues within the amino acid sequences of the antibody. Any combination of deletion, insertion, and substitution can be made to arrive at the final construct, provided that the final construct possesses the desired characteristics. The amino acid alterations may be introduced in the subject antibody amino acid sequence at the time that sequence is made.

A useful method for identification of certain residues or regions of the antibody that are preferred locations for mutagenesis is called "alanine scanning mutagenesis" as described by Cunningham and Wells (1989) Science, 244:1081-1085. Here, a residue or group of target residues are identified (*e.g.*, charged residues such as arg, asp, his, lys, and glu) and replaced by a neutral or negatively charged amino acid (*e.g.*, alanine or polyalanine) to affect the interaction of the amino acids with antigen. Those amino acid locations demonstrating functional sensitivity to the substitutions then are refined by introducing further or other modifications at, or for, the sites of substitution. Thus, while the site for introducing an amino acid sequence modification is predetermined, the nature of the mutation *per se* need not be predetermined. For example, to analyze the performance of a mutation at a given site, ala scanning or random mutagenesis is conducted at the target codon or region and the expressed immunoglobulins are screened for the desired activity.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue. Other insertional modifications of the antibody molecule include the fusion to the N- or C-terminus of the antibody to an enzyme (*e.g.* for ADEPT) or a polypeptide which increases the serum half-life of the antibody.

In certain embodiments, variant IgG of the present invention is altered to increase or decrease the extent to which the antibody is glycosylated. Glycosylation of polypeptides is typically either N-linked or O-linked. N-linked refers to the attachment of a carbohydrate moiety to the side chain of an asparagine residue. The tripeptide sequences asparagine-X-serine and asparagine-X-threonine, where X is any amino acid except proline, are the recognition sequences for enzymatic attachment of the carbohydrate moiety to the asparagine side chain. Thus, the presence of either of these tripeptide sequences in a polypeptide creates a potential glycosylation site. O-linked glycosylation refers to the attachment of one of the sugars N-aceylgalactosamine, galactose, or xylose to a hydroxyamino acid, most commonly serine or threonine, although 5-hydroxyproline or 5-hydroxylysine may also be used.

Addition or deletion of glycosylation sites to the antibody is conveniently accomplished by altering the amino acid sequence such that one or more of the above-described tripeptide sequences (for N-linked glycosylation sites) is created or removed. The alteration may also be made by the addition, deletion, or substitution of one or more serine or threonine residues to the sequence of the original antibody (for O-linked glycosylation sites).

The carbohydrate attached to the Fc region of the variant IgGs may be altered. Native antibodies produced by mammalian cells typically comprise a branched, biantennary oligosaccharide that is generally attached by an N-linkage to Asn297 of the CH2 domain of the Fc region. *See, e.g.,* Wright et al. (1997) TIBTECH 15:26-32. The oligosaccharide may include various carbohydrates, *e.g.*, mannose, N-acetyl glucosamine (GlcNAc), galactose, and sialic acid, as well as a fucose attached to a GlcNAc in the "stem" of the biantennary oligosaccharide structure. In some embodiments, modifications of the oligosaccharide in a variant IgG of the invention may be made in order to create variant IgGs with certain additionally improved properties.

For example, antibody modifications are provided having a carbohydrate structure that lacks fucose attached (directly or indirectly) to an Fc region. Such modifications may have improved ADCC function. *See, e.g.,* US Patent Publication Nos. US 2003/0157108 (Presta, L.); US 2004/0093621 (Kyowa Hakko Kogyo Co., Ltd). Examples of publications related to "defucosylated" or "fucose-deficient" antibody modifications include: US 2003/0157108; WO 2000/61739; WO 2001/29246; US 2003/0115614; US 2002/0164328; US 2004/0093621; US 2004/0132140; US 2004/0110704; US 2004/0110282; US 2004/0109865; WO 2003/085119; WO 2003/084570; WO 2005/035586; WO 2005/035778; WO2005/053742; WO2002/031140; Okazaki et al. J. Mol. Biol. 336:1239-1249 (2004); Yamane-Ohnuki et al. Biotech. Bioeng. 87: 614 (2004). Examples of cell lines capable of producing defucosylated antibodies include Lec13 CHO cells deficient in protein fucosylation (Ripka et al. Arch. Biochem. Biophys. 249:533-545 (1986); US Pat Appl No US 2003/0157108 A1, Presta, L; and WO 2004/056312 A1, Adams et al., especially at Example 11), and knockout cell lines, such as alpha-1,6-fucosyltransferase gene, *FUT8,* knockout CHO cells *(see, e.g.,* Yamane-Ohnuki et al. Biotech. Bioeng. 87: 614 (2004); Kanda, Y. et al., Biotechnol. Bioeng., 94(4):680-688 (2006); and WO2003/085107).

Antibody modifications are further provided with bisected oligosaccharides, *e.g.*, in which a biantennary oligosaccharide attached to the Fc region of the antibody is bisected by GlcNAc. Such antibody variants may have reduced fucosylation and/or improved ADCC function. Examples of such antibody modifications are described, *e.g.,* in WO 2003/011878 (Jean-Mairet et al.); US Patent No. 6,602,684 (Umaña et al.); and US 2005/0123546 (Umaña et al.). Antibody modifications with at least one galactose residue in the oligosaccharide attached to the Fc region are also provided. Such antibody modifications may have improved CDC function. Such antibody modifications are described, *e.g.,* in WO 1997/30087 (Patel et al.); WO 1998/58964 (Raju, S.); and WO 1999/22764 (Raju, S.).

In certain embodiments, the invention contemplates an antibody modifications that possesses some but not all effector functions, which make it a desirable candidate for many applications in which the half life of the antibody *in vivo* is important yet certain effector functions (such as complement and ADCC) are unnecessary or deleterious. In certain embodiments, the Fc activities of the antibody are measured to ensure that only the desired properties are maintained. *In vitro* and/or *in vivo* cytotoxicity assays can be conducted to confirm the reduction/depletion of CDC and/or ADCC activities. For example, Fc receptor (FcR) binding assays can be conducted to ensure that the antibody lacks FcyR binding (hence likely lacking ADCC activity), but retains FcRn binding ability. The primary cells for mediating ADCC, NK cells, express Fc(RIII only, whereas monocytes express Fc(RI, Fc(RII and Fc(RIII. FcR expression on hematopoietic cells is summarized in Table 3 on page 464 of Ravetch and Kinet, Annu. Rev. Immunol. 9:457-92 (1991). Non-limiting examples of *in vitro* assays to assess ADCC activity of a molecule of interest is described in U.S. Patent No. 5,500,362 (*see, e.g.* Hellstrom, I., et al. Proc. Nat'l Acad. Sci. USA 83:7059-7063 (1986)) and Hellstrom, I et al., Proc. Nat'l Acad. Sci. USA 82:1499-1502 (1985); 5,821,337 (*see* Bruggemann, M. et al., J. Exp. Med. 166:1351-1361 (1987)). Alternatively, non-radioactive assays methods may be employed (*see,* for example, ACTI™ non-radioactive cytotoxicity assay for flow cytometry (CellTechnology, Inc. Mountain View, CA); and CytoTox 96® non-radioactive cytotoxicity assay (Promega, Madison, WI)). Useful effector cells for such assays include peripheral blood mononuclear cells (PBMC) and Natural Killer (NK) cells. Alternatively, or additionally, ADCC activity of the molecule of interest may be assessed *in vivo, e.g.,* in a animal model such as that disclosed in Clynes et al. Proc. Nat'l Acad. Sci. USA 95:652-656 (1998). C1q binding assays may also be carried out to confirm that the antibody is unable to bind C1q and hence lacks CDC activity. To assess complement activation, a CDC assay may be performed (see, for example, Gazzano-Santoro et al., J. Immunol. Methods 202:163 (1996); Cragg, M.S. et al., Blood 101:1045-1052 (2003); and Cragg, M.S. and M.J. Glennie, Blood 103:2738-2743 (2004)). FcRn binding and *in vivo* clearance/half life determinations can also be performed using methods known in the art *(see,* for example, Petkova, S.B. et al., Int'l. Immunol. 18(12):1759-1769 (2006)).

Other antibody modifications having one or more amino acid substitutions are provided. Sites of interest for substitutional mutagenesis include the hypervariable regions, but FR alterations are also contemplated. Conservative substitutions are shown in Table 1 under the heading of "preferred substitutions." More substantial changes, denominated "exemplary substitutions" are provided in Table 1, or as further described below in reference to amino acid classes. Amino acid substitutions may be introduced into an antibody of interest and the products screened, *e.g.,* for a desired activity, such as improved antigen binding, decreased immunogenicity, improved ADCC or CDC, *etc.*

***TABLE 1***

| Original Residue | Exemplary Substitutions | Preferred Substitutions |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |

(continued)

| Original Residue | Exemplary Substitutions | Preferred Substitutions |
|---|---|---|
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Modifications in the biological properties of an antibody may be accomplished by selecting substitutions that affect (a) the structure of the polypeptide backbone in the area of the substitution, for example, as a sheet or helical conformation, (b) the charge or hydrophobicity of the molecule at the target site, or (c) the bulk of the side chain. Amino acids may be grouped according to similarities in the properties of their side chains (in A. L. Lehninger, in Biochemistry, second ed., pp. 73-75, Worth Publishers, New York (1975)):

(1) non-polar: Ala (A), Val (V), Leu (L), Ile (I), Pro (P), Phe (F), Trp (W), Met (M)
(2) uncharged polar: Gly (G), Ser (S), Thr (T), Cys (C), Tyr (Y), Asn (N), Gln (Q)
(3) acidic: Asp (D), Glu (E)
(4) basic: Lys (K), Arg (R), His(H)

Alternatively, naturally occurring residues may be divided into groups based on common side-chain properties:

(1) hydrophobic: Norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

Non-conservative substitutions will entail exchanging a member of one of these classes for another class. Such substituted residues also may be introduced into the conservative substitution sites or, into the remaining (non-conserved) sites.

One type of substitutional modification involves substituting one or more hypervariable region residues of a parent antibody (e.g. a humanized or human antibody). In certain embodiments, the parent antibody is the wild-type counterpart variant IgG *(e.g.,* a variant IgG of the invention without any additional alteration in its amino acid sequence). Generally, the resulting antibodies selected for further development will have modified *(e.g.,* improved) biological properties relative to the parent antibody from which they are generated. An exemplary substitutional modifcation is an affinity matured antibody, which may be conveniently generated using phage display-based affinity maturation techniques. Briefly, several hypervariable region sites (*e.g.* 6-7 sites) are mutated to generate all possible amino acid substitutions at each site. The antibodies thus generated are displayed from filamentous phage particles as fusions to at least part of a phage coat protein (*e.g.,* the gene III product of M13) packaged within each particle. The phage-displayed antibodies are then screened for their biological activity (*e.g.* binding affinity). In order to identify candidate hypervariable region sites for modification, scanning mutagenesis (*e.g.*, alanine scanning) can be performed to identify hypervariable region residues contributing significantly to antigen binding. Alternatively, or additionally, it may be beneficial to analyze a crystal structure of the antigen-antibody complex to identify contact points between the antibody and antigen. Such contact residues and neighboring residues are candidates for substitution according to techniques known in the art, including those elaborated herein. Once such modified antibodies are generated, the panel of antibodies is subjected to screening using techniques known in the art, including those described herein, and antibodies with superior properties in one or more relevant assays may be selected for further development.

Nucleic acid molecules encoding amino acid sequence of the modified antibody (*e.g.*, modified variant IgG) are prepared by a variety of methods known in the art. These methods include, but are not limited to, isolation from a natural source (in the case of naturally occurring amino acid sequence modifications) or preparation by oligonucleotide-mediated (or site-directed) mutagenesis, PCR mutagenesis, and cassette mutagenesis of an earlier prepared modified antibody or a non-modified version of the antibody.

In accordance with this description and the teachings of the art, it is contemplated that in certain embodiments, an antibody modification of the invention may comprise one or more alterations as compared to the wild-type counterpart variant IgG (*e.g.,* a variant IgG of the invention without any additional alteration in its amino acid sequence). These antibody modifications comprising additional alterations would nonetheless retain substantially the same characteristics required for therapeutic utility as compared to the wild-type counterpart variant IgG.

In another aspect, the invention provides antibody modifications comprising modifications in the interface of Fc polypeptides comprising the Fc region, wherein the modifications facilitate and/or promote heterodimerization. These modifications comprise introduction of a protuberance into a first Fc polypeptide and a cavity into a second Fc polypeptide, wherein the protuberance is positionable in the cavity so as to promote complexing of the first and second Fc polypeptides. Methods of generating antibodies with these modifications are known in the art, *e.g.,* as described in U.S. Pat. No. 5,731,168.

In yet another aspect, it may be desirable to create cysteine engineered antibodies, *e.g.*, "thioMAbs," in which one or more residues of an antibody are substituted with cysteine residues. In certain embodiments, the substituted residues occur at accessible sites of the antibody. By substituting those residues with cysteine, reactive thiol groups are thereby positioned at accessible sites of the antibody and may be used to conjugate the antibody to other moieties, such as drug moieties or linker-drug moieties, as described further herein. In certain embodiments, any one or more of the following residues may be substituted with cysteine: V205 (Kabat numbering) of the light chain; A118 (EU numbering) of the heavy chain; and S400 (EU numbering) of the heavy chain Fc region.

### *Antibody Derivatives*

In certain embodiments, the variant IgGs of the present invention can be further modified to contain additional nonproteinaceous moieties that are known in the art and readily available. In certain embodiments, the variant IgG may be conjugated with a cytotoxic agent. In certain embodiments, the variant IgG to which the cytotoxic agent is bound is internalized by the cell, resulting in increased therapeutic efficacy of the conjugate in killing the cell to which it binds.

In certain embodiments, the moieties suitable for derivatization of the antibody are water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1, 3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, propropylene glycol homopolymers, prolypropylene oxide/ethylene oxide copolymers, polyoxyethylated polyols (*e.g.,* glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody may vary, and if more than one polymer are attached, they can be the same or different molecules. In general, the number and/or type of polymers used for derivatization can be determined based on considerations including, but not limited to, the particular properties or functions of the antibody to be improved, whether the antibody derivative will be used in a therapy under defined conditions, etc.

In another embodiment, conjugates of an antibody and nonproteinaceous moiety that may be selectively heated by exposure to radiation are provided. In one embodiment, the nonproteinaceous moiety is a carbon nanotube (Kam et al., Proc. Natl. Acad. Sci. USA 102: 11600-11605 (2005)). The radiation may be of any wavelength, and includes, but is not limited to, wavelengths that do not harm ordinary cells, but which heat the nonproteinaceous moiety to a temperature at which cells proximal to the antibody-nonproteinaceous moiety are killed.

### *Making Variant IgGs*

The variant IgGs can be made by any method known in the art. In certain embodiments, the variant IgG sequences are used to create nucleic acids that encode the member sequences, and that may then be cloned into host cells, expressed and assayed, if desired. These practices are carried out using well-known procedures, and a variety of methods that may find use in are described in Molecular Cloning--A Laboratory Manual, 3rd Ed. (Maniatis, Cold Spring Harbor Laboratory Press, New York, 2001), and Current Protocols in Molecular Biology (John Wiley & Sons). The nucleic acids that encode the variant IgGs may be incorporated into an expression vector in order to express the protein. Expression vectors typically include a protein operably linked, that is, placed in a functional relationship, with control or regulatory sequences, selectable markers, any fusion partners, and/or additional elements. The variant IgGs may be produced by culturing a host cell transformed with nucleic acid, preferably an expression vector, containing nucleic acid encoding the variant IgGs, under the appropriate conditions to induce or cause expression of the protein. A wide variety of appropriate host cells may be used, including but not limited to mammalian cells, bacteria, insect cells, and yeast. For example, a variety of cell lines that may find use are described in the ATCC cell line catalog, available from the American Type Culture Collection. The methods of introducing exogenous nucleic acid into host cells are well known in the art, and will vary with the host cell used.

In certain embodiments, variant IgGs are purified or isolated after expression. Antibodies may be isolated or purified in a variety of ways known to those skilled in the art. Standard purification methods include chromatographic techniques, electrophoretic, immunological, precipitation, dialysis, filtration, concentration, and chromatofocusing techniques. As is well known in the art, a variety of natural proteins bind antibodies, for example certain bacterial proteins, and these proteins may find use in purification. Often, purification may be enabled by a particular fusion partner. For example, proteins may be purified using glutathione resin if a GST fusion is employed, $Ni^{+2}$ affinity chromatography if a His-tag is employed, or immobilized anti-flag antibody if a flag-tag is used. For general guidance in suitable purification techniques, *see* Antibody Purification: Principles and Practice, 3rd Ed., Scopes, Springer-Verlag, NY, 1994.

### *Screening Variant IgGs*

Variant IgGs of the present invention may be screened using a variety of methods, including but not limited to those that use *in vitro* assays, *in vivo* and cell-based assays, and selection technologies. Automation and high-throughput screening technologies may be utilized in the screening procedures. Screening may employ the use of a fusion partner or label, for example an immune label, isotopic label, or small molecule label such as a fluorescent or calorimetric dye.

In certain embodiment, the functional and/or biophysical properties of variant IgGs are screened in an *in vitro* assay. In certain embodiments, the protein is screened for functionality, for example its ability to catalyze a reaction or its binding affinity to its target.

A subset of screening methods are those that select for favorable members of a library. The methods are herein referred to as "selection methods," and these methods find use in the present invention for screening variant IgGs. When protein libraries are screened using a selection method, only those members of a library that are favorable, that is which meet some selection criteria, are propagated, isolated, and/or observed. A variety of selection methods are known in the art that may find use in the present invention for screening protein libraries. Other selection methods that may find use in the present invention include methods that do not rely on display, such as in vivo methods. A subset of selection methods referred to as "directed evolution" methods are those that include the mating or breading of favorable sequences during selection, sometimes with the incorporation of new mutations.

In certain embodiments, variant IgGs are screened using one or more cell-based or *in vivo* assays. For such assays, purified or unpurified proteins are typically added exogenously such that cells are exposed to individual variants or pools of variants belonging to a library. These assays are typically, but not always, based on the function of the variant IgG; that is, the ability of the variant IgG to bind to its target and mediate some biochemical event, for example effector function, ligand/receptor binding inhibition, apoptosis, and the like. Such assays often involve monitoring the response of cells to the IgG, for example cell proliferation, cell migration, angiogenesis, cell survival, cell death, change in cellular morphology, or transcriptional activation such as cellular expression of a natural gene or reporter gene. For example, such assays may measure the ability of IgG variants to elicit ADCC, ADCP, or CDC. For some assays additional cells or components, that is in addition to the target cells, may need to be added, for example serum complement, or effector cells such as peripheral blood monocytes (PBMCs), NK cells, macrophages, and the like. Such additional cells may be from any organism, preferably humans, mice, rat, rabbit, and monkey. In certain embodiments, antibodies may inhibit angiogenesis and methods for monitoring such activity are well known in the art. In yet another embodiment, antibodies may cause apoptosis of certain cell lines expressing the target, or they may mediate attack on target cells by immune cells which have been added to the assay. Methods for monitoring cell death or viability are known in the art, and include the use of dyes, immunochemical, cytochemical, and radioactive reagents. Transcriptional activation may also serve as a method for assaying function in cell-based assays. Alternatively, cell-based screens are performed using cells that have been transformed or transfected with nucleic acids encoding the variants. That is, variant IgGs are not added exogenously to the cells.

The biological properties of the variant IgGs may be characterized in cell, tissue, and whole organism experiments. Drugs are often tested in animals, including but not limited to mice, rats, rabbits, dogs, cats, pigs, and monkeys, in order to measure a drug's efficacy for treatment against a disease or disease model, or to measure a drug's pharmacokinetics, toxicity, and other properties. The animals may be referred to as disease models. Therapeutics are often tested in mice, including but not limited to nude mice, SCID mice, xenograft mice, and transgenic mice (including knockins and knockouts). Such experimentation may provide meaningful data for determination of the potential of the protein to be used as a therapeutic. Any organism, preferably mammals, may be used for testing. For example because of their genetic similarity to humans, monkeys can be suitable therapeutic models, and thus may be used to test the efficacy, toxicity, pharmacokinetics, or other property of the variant IgGs. Tests of the in humans are ultimately required for approval as drugs, and thus of course these experiments are contemplated. Thus the variant IgGs may be tested in humans to determine their therapeutic efficacy, toxicity, immunogenicity, pharmacokinetics, and/or other clinical properties.

### *Therapeutic Uses of Variant IgGs*

The variant IgGs may find use in a wide range of products. In certain embodiments the IgG variant is a therapeutic, a diagnostic, or a research reagent. The variant IgG may find use in an antibody composition that is monoclonal or polyclonal.

The variant IgGs may be used for various therapeutic purposes, including but not limited to treating patients with *S. aureus* infections.

### *Dosages, Formulations, and Duration*

The variant IgG composition will be formulated, dosed, and administered in a fashion consistent with good medical practice. Factors for consideration in this context include, but not limited to, the particular disorder being treated, the particular mammal being treated, the clinical condition of the individual patient, the cause of the disorder, the site of delivery of the agent, the method of administration, the scheduling of administration, and other factors known to medical practitioners. For the prevention or treatment of disease, the appropriate dosage of a variant IgG, *e.g.,* an antibody, of the invention (when used alone or in combination with one or more other additional therapeutic agents) will depend on the type of disease to be treated, the type of antibody, the severity and course of the disease, whether the antibody is administered for preventive or therapeutic purposes, previous therapy, the patient's clinical history and response to the antibody, and the discretion of the attending physician. The variant IgG is suitably administered to the patient at one time or over a series of treatments.

Pharmaceutical formulations herein may also contain more than one active compound as necessary for the particular indication being treated, preferably those with complementary activities that do not adversely affect each other. Such molecules are suitably present in combination in amounts that are effective for the purpose intended.

For the prevention or treatment of a disease, the appropriate dosage of a variant IgG of the invention (when used alone or in combination with one or more other additional therapeutic agents) will depend on the type of disease to be treated, the type of antibody, the severity and course of the disease, whether the variant IgG is administered for preventive or therapeutic purposes, previous therapy, the patient's clinical history and response to the variant IgG, and the discretion of the attending physician. In certain embodiments, the variant IgG is suitably administered to the patient at one time or over a series of treatments. Depending on the type and severity of the disease, about 1 μg/kg to 20 mg/kg (*e.g.,* 0.1mg/kg-15mg/kg) of variant IgG can be an initial candidate dosage for administration to the patient, whether, for example, by one or more separate administrations, or by continuous infusion. One typical daily dosage might range from about 1 μg/kg to 100 mg/kg or more, depending on the factors mentioned above. For repeated administrations over several days or longer, depending on the condition, the treatment would generally be sustained until a desired suppression of disease symptoms occurs. In one embodiment, depending on the condition, the treatment is sustained until the disease is treated, as measured by the methods described herein or known in the art. One exemplary dosage of the variant IgG would be in the range from about 0.05 mg/kg to about 20 mg/kg. Thus, one or more doses of about 0.5 mg/kg, 2.0 mg/kg, 4.0 mg/kg, 7.5 mg/kg, 10 mg/kg or 15 mg/kg (or any combination thereof) may be administered to the patient. Such doses may be administered intermittently, *e.g.*, every three, every eight or every twelve weeks (*e.g.,* such that the patient receives from about two to about twenty, or *e.g.,* about six doses of the antibody). In certain embodiments, an initial higher loading dose, followed by one or more lower doses may be administered. In certain embodiments, dosing regimen comprises administering an initial loading dose of about 4 mg/kg, followed by a weekly maintenance dose of about 2 mg/kg of the antibody. However, other dosage regimens may be useful. The progress of this therapy is easily monitored by conventional techniques and assays.

In certain embodiments, the patient is treated with a combination of the variant IgG and one or more other therapeutic agent(s). The combined administration includes coadministration or concurrent administration, using separate formulations or a single pharmaceutical formulation, and consecutive administration in either order, wherein optionally there is a time period while both (or all) active agents simultaneously exert their biological activities. The effective amounts of therapeutic agents administered in combination with a variant IgG will be at the physicians's or veterinarian's discretion. Dosage administration and adjustment is done to achieve maximal management of the conditions to be treated. The dose will additionally depend on such factors as the type of therapeutic agent to be used and the specific patient being treated. In certain embodiments, the combination of the inhibitors potentiates the efficacy of a single inhibitor. The term "potentiate" refers to an improvement in the efficacy of a therapeutic agent at its common or approved dose.

Variant IgG of the invention (and any additional therapeutic agent) can be administered by any suitable means, including parenteral, subcutaneous, intraperitoneal, intracerobrospinal, intrapulmonary, and intranasal, and, if desired for local treatment, intralesional administration. Parenteral infusions include intramuscular, intravenous, intraarterial, intraperitoneal, or subcutaneous administration. In certain embodiments, the variant IgG, *e.g.,* an antibody, is suitably administered by pulse infusion, particularly with declining doses of the variant IgG. Dosing can be by any suitable route, *e.g.* by injections, such as intravenous or subcutaneous injections, depending in part on whether the administration is brief or chronic. In certain embodiments, the variant IgG is administered to a subject intravenously, *e.g*., as a bolus or by continuous infusion over a period of time.

The location of the binding target of a variant IgG, *e.g.,* an antibody, of the invention may be taken into consideration in preparation and administration of the variant IgG. When the binding target of a variant IgG is located in the brain, certain embodiments of the invention provide for the variant IgG to traverse the blood-brain barrier. Several art-known approaches exist for transporting molecules across the blood-brain barrier, including, but not limited to, physical methods, lipid-based methods, stem cell-based methods, and receptor and channel-based methods.

Physical methods of transporting a variant IgG, *e.g.,* an antibody, across the blood-brain barrier include, but are not limited to, circumventing the blood-brain barrier entirely, or by creating openings in the blood-brain barrier. Circumvention methods include, but are not limited to, direct injection into the brain (*see, e.g.,* Papanastassiou et al., Gene Therapy 9: 398-406 (2002)), interstitial infusion/convection-enhanced delivery (see, e.g., Bobo et al., Proc. Natl. Acad. Sci. USA 91: 2076-2080 (1994)), and implanting a delivery device in the brain *(see, e.g.,* Gill et al., Nature Med. 9: 589-595 (2003); and Gliadel Wafers™, Guildford Pharmaceutical). Methods of creating openings in the barrier include, but are not limited to, ultrasound (*see, e.g.,* U.S. Patent Publication No. 2002/0038086), osmotic pressure (*e.g.,* by administration of hypertonic mannitol (Neuwelt, E. A., Implication of the Blood-Brain Barrier and its Manipulation, Vols 1 & 2, Plenum Press, N.Y. (1989)), permeabilization by, *e.g.,* bradykinin or permeabilizer A-7 *(see, e.g.,* U.S. Patent Nos. 5,112,596, 5,268,164, 5,506,206, and 5,686,416), and transfection of neurons that straddle the blood-brain barrier with vectors containing genes encoding the variant IgG *(see, e.g.,* U.S. Patent Publication No. 2003/0083299).

Lipid-based methods of transporting a variant IgG, *e.g.,* an antibody, across the blood-brain barrier include, but are not limited to, encapsulating the variant IgG in liposomes that are coupled to antibody binding fragments that bind to receptors on the vascular endothelium of the blood-brain barrier *(see, e.g.,* U.S. Patent Application Publication No. 20020025313), and coating the variant IgG in low-density lipoprotein particles (*see, e.g.,* U.S. Patent Application Publication No. 20040204354) or apolipoprotein E (*see, e.g.,* U.S. Patent Application Publication No. 20040131692).

Stem-cell based methods of transporting a variant IgG, *e.g.,* an antibody, across the blood-brain barrier entail genetically engineering neural progenitor cells (NPCs) to express the antibody of interest and then implanting the stem cells into the brain of the individual to be treated. *See* Behrstock et al. (2005) Gene Ther. 15 Dec. 2005 advanced online publication (reporting that NPCs genetically engineered to express the neurotrophic factor GDNF reduced symptoms of Parkinson disease when implanted into the brains of rodent and primate models).

Receptor and channel-based methods of transporting a variant IgG, *e.g.,* an antibody, across the blood-brain barrier include, but are not limited to, using glucocorticoid blockers to increase permeability of the blood-brain barrier (*see, e.g.,* U.S. Patent Application Publication Nos. 2002/0065259, 2003/0162695, and 2005/0124533); activating potassium channels (see, e.g., U.S. Patent Application Publication No. 2005/0089473), inhibiting ABC drug transporters (*see, e.g.,* U.S. Patent Application Publication No. 2003/0073713); coating antibodies with a transferrin and modulating activity of the one or more transferrin receptors (*see, e.g.,* U.S. Patent Application Publication No. 2003/0129186), and cationizing the antibodies (*see, e.g.,* U.S. Patent No. 5,004,697).

Pharmaceutical formulations comprising a variant IgG, *e.g.,* an antibody, of the invention are prepared for storage by mixing the variant IgG having the desired degree of purity with optional physiologically acceptable carriers, excipients or stabilizers (Remington: The Science and Practice of Pharmacy 20th edition (2000)), in the form of aqueous solutions, lyophilized or other dried formulations. Acceptable carriers, excipients, or stabilizers are nontoxic to recipients at the dosages and concentrations employed, and include buffers such as phosphate, citrate, histidine and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (*e.g.*, Zn-protein complexes); and/or non-ionic surfactants such as TWEEN™, PLURONICS™ or polyethylene glycol (PEG).

The active ingredients may also be entrapped in microcapsule prepared, for example, by coacervation techniques or by interfacial polymerization, for example, hydroxymethylcellulose or gelatin-microcapsule and poly-(methylmethacylate) microcapsule, respectively, in colloidal drug delivery systems (for example, liposomes, albumin microspheres, microemulsions, nano-particles and nanocapsules) or in macroemulsions. Such techniques are disclosed in Remington: The Science and Practice of Pharmacy 20th edition (2000).

The formulations to be used for *in vivo* administration must be sterile. This is readily accomplished, e.g. by filtration through sterile filtration membranes.

Sustained-release preparations may be prepared. Suitable examples of sustained-release preparations include semipermeable matrices of solid hydrophobic polymers containing the immunoglobulin of the invention, which matrices are in the form of shaped articles, e.g., films, or microcapsule. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and $\gamma$ ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated immunoglobulins remain in the body for a long time, they may denature or aggregate as a result of exposure to moisture at 37°C, resulting in a loss of biological activity and possible changes in immunogenicity. Rational strategies can be devised for stabilization depending on the mechanism involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, and developing specific polymer matrix compositions.

### *Combination Therapies*

Therapeutics described herein may be administered with other therapeutics concomitantly, *i.e.,* the therapeutics described herein may be co-administered with other therapies or therapeutics, including for example, small molecules, other biologicals, radiation therapy, surgery, *etc.*

### *Articles of Manufacture*

In another aspect of the invention, an article of manufacture containing materials useful for the treatment, prevention and/or diagnosis of the disorders described above is provided. The article of manufacture comprises a container and a label or package insert on or associated with the container. Suitable containers include, for example, bottles, vials, syringes, *etc.* The containers may be formed from a variety of materials such as glass or plastic. The container holds a composition which is by itself or combined with another composition effective for treating, preventing and/or diagnosing the condition and may have a sterile access port (for example the container may be an intravenous solution bag or a vial having a stopper pierceable by a hypodermic injection needle). The label or package insert indicates that the composition is used for treating the condition of choice. In certain embodiments, the article of manufacture may comprise (a) a first container with a composition contained therein, wherein the composition comprises a variant IgG of the invention; and (b) a second container with a composition contained therein, wherein the composition comprises a further therapeutic agent. The article of manufacture may further comprise a package insert indicating that the compositions can be used to treat a particular condition. Alternatively, or additionally, the article of manufacture may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

In certain embodiments, the variant IgG can be packaged alone or in combination with other therapeutic compounds as a kit. The kit can include optional components that aid in the administration of the unit dose to patients, such as vials for reconstituting powder forms, syringes for injection, customized IV delivery systems, inhalers, *etc.* Additionally, the unit dose kit can contain instructions for preparation and administration of the compositions. The kit may be manufactured as a single use unit dose for one patient, multiple uses for a particular patient (at a constant dose or in which the individual compounds may vary in potency as therapy progresses); or the kit may contain multiple doses suitable for administration to multiple patients ("bulk packaging"). The kit components may be assembled in cartons, blister packs, bottles, tubes, and the like.

## EXAMPLES

The following are examples of methods and compositions of the invention. It is understood that various other embodiments may be practiced, given the general description provided above.

### Example 1: Production of Anti-Her2 Variants

The Fab regions of humanized anti-Her2 (4D5 or huMAb4D5-8 described in U.S. Patent No. 5,821,337) $IgG_1$ heavy and light were cloned separately into two pRK-based transient transfection plasmids containing human $IgG_1$ constant domains Kunkel based site-directed mutagenesis was then used to generate anti-Her2 $IgG_1$ variants in which

residues in the $V_H$ domain were mutated. The anti-Her2 variants generated in this study were S17A, R19A, S21A, T57A, T57K, R66A, T68A, S70A, Y79A, Q81A, N82aA, and S82bA (all numbered according to the EU index as in Kabat).

Plasmids containing the variants' heavy chain and wild-type light chain were co-transfected into the adenovirus-transformed human embryonic kidney cell line 293 using FUGENE® (Roche, Basel, Switzerland) according to the manufacturing protocol. After 24 hours of incubation with the transfection complexes, transfected cell were cultured with serum free media 1.3x GEM N Medium with 5mM glutamine. Supernatants were collected, and conditioned with 1M TRIS pH 8.0 and 5M sodium chloride (NaCl) to give a final concentration of 30mM TRIS and 50mM NaCl. Conditioned supernatant were then loaded onto a Protein L resin-packed column (Thermo scientific, Rockford, IL). After loading, the column was washed with buffer containing 30mM TRIS and 150mM NaCl pH 8. Bound Fab was eluted with 0.1M glycine buffer pH 3.0. Next, purified Fab were concentrated and injected over a Superdex®-200 size exclusion chromatography column (GE healthcare, Chalfont St. Giles, United Kingdom) to remove any aggregates. Monomeric fractions were pooled together and used for the binding studies. Anti-HER2 wild-type and anti-HER2 variant Fab concentrations were calculated using absorbance reading at 280nM, and an absorbance of 1.5 was estimated to be 1mg/ml of Fab.

Example 2: Protein A Binding Studies

The binding of anti-Her2 variants to protein A were studied by ELISA. MaxiSorp™ ELISA plates (Thermo scientific, Rockford, IL) were coated overnight with either 1μg/ml of protein A (Thermo scientific, Rockford, IL) or the extracellular domain of Her2 (Genentech, South San Francisco, CA). Plates were blocked with PBS, 0.5% BSA, 10ppm Proclin, pH 7.2 for 1 hr at room temperature and then washed with wash buffer (PBS/0.05% Tween™ 20/pH 7.2). Serial 4-fold dilutions (starting at 1000 nM) of the wild-type Fab and variants in assay buffer (PBS, pH 7.4, 0.5% BSA, 0.05% Tween 20, 10ppm Proclin) were added to the 96 well plates coated with protein A. Meanwhile, serial 4-fold dilutions (starting at 50 nM) of the wild-type Fab and variants in assay buffer were added to the 96 well plates coated with Her2. After three hours of incubation at room temperature with shaking, plates were washed 4 times, and bound antibody was detected with goat anti-human IgG ($F(ab')_2$ specific)-HRP (Jackson ImmunoResearch, West Grove, PA) diluted 1:10,000 in assay buffer for 0.5 hr at room temperature with shaking. Plates were then washed 4 times again, followed by the addition of tetramethyl benzidine substrate (Moss, Pasadena, MD) for color development. The reaction was stopped after 2 minutes by the addition of 1M phosphoric acid ($H_3PO_4$). Plates were read on a Molecular Devices microplate reader at a wavelength of 450-620 nm.

Results show that all of the variants retained the same binding affinity to Her2 as wild-type (Figures 1 and 2). Results in Figures 3-4 show that certain variants show reduced binding to protein A relative to wild-type [S17A, R19A, T57A, T57K, R66A, Q81A, and N82aA], whereas others show essentially the same level of binding [S21A and T68A] and still others show increased binding [S70A, Y79A, and S82bA]. The EC 50 of WT Fab was estimated to be about 15nM. Estimates of EC50 for each of these variants are shown in Table 2.

Table 2. Binding of variants to protein A

| Fab variant | EC 50 (nM) |
|---|---|
| S17A | **310** |
| R19A | **>10,000** |
| S21A | **16** |
| T57A | **>10,000** |
| T57K | **900** |
| R66A | **> 5,000** |
| T68A | **22** |
| S70A | **1.6** |
| Y79A | **6** |
| Q81A | **970** |
| N82aA | **>10,000** |
| S82bA | **1.3** |

Although the foregoing invention has been described in some detail by way of illustration and example for purposes of clarity of understanding, the descriptions and examples should not be construed as limiting the scope of the invention.

## Claims

1. A variant IgG comprising a human IgG $V_H$ region comprising one or more amino acid substitutions relative to a wild-type human IgG $V_H$ region at one or more of amino acid residues 17, 19, 57, 66, 70, 79, 81, 82a, 82b, numbered according to the EU index as in Kabat, wherein the variant IgG has altered binding to *Staphylococcus aureus* protein A.

2. The variant IgG of claim 1, which has increased binding to protein A.

3. The variant IgG of claim 2, wherein the variant IgG comprises a human IgG $V_H$ region comprising one or more amino acid substitutions relative to a wild-type IgG $V_H$ region at one or more of amino acid residues 70, 79, and 82b.

4. The variant IgG of claim 3, wherein said one or more amino acid substitutions are selected from the group consisting of S70A, Y79A, and S82bA.

5. The variant IgG of claim 1, which has decreased binding to protein A.

6. The variant IgG of claim 5, wherein the variant IgG comprises a human IgG $V_H$ region comprising one or more amino acid substitutions relative to a wild-type IgG $V_H$ region at one or more of amino acid residues 17, 19, 57, 66, 81, and 82a.

7. The variant IgG of claim 6, wherein said one or more amino acid substitutions are selected from the group consisting of S17A, R19A, T57A, T57K, R66A, Q81A, and N82aA.

8. The variant IgG of claim 1 which is a human or humanized IgG.

9. The variant IgG of claim 8 which is $IgG_1$, $IgG_2$, $IgG_3$ or $IgG_4$.

10. The variant IgG of claim 1, wherein the variant IgG binds to a *Staphylococcus aureus* protein other than protein A.

11. A pharmaceutical composition comprising the variant IgG of claim 1 or 10 and a pharmaceutically acceptable carrier.

12. A kit comprising the variant IgG of claim 1 or 10, in a container, and instructions for use.

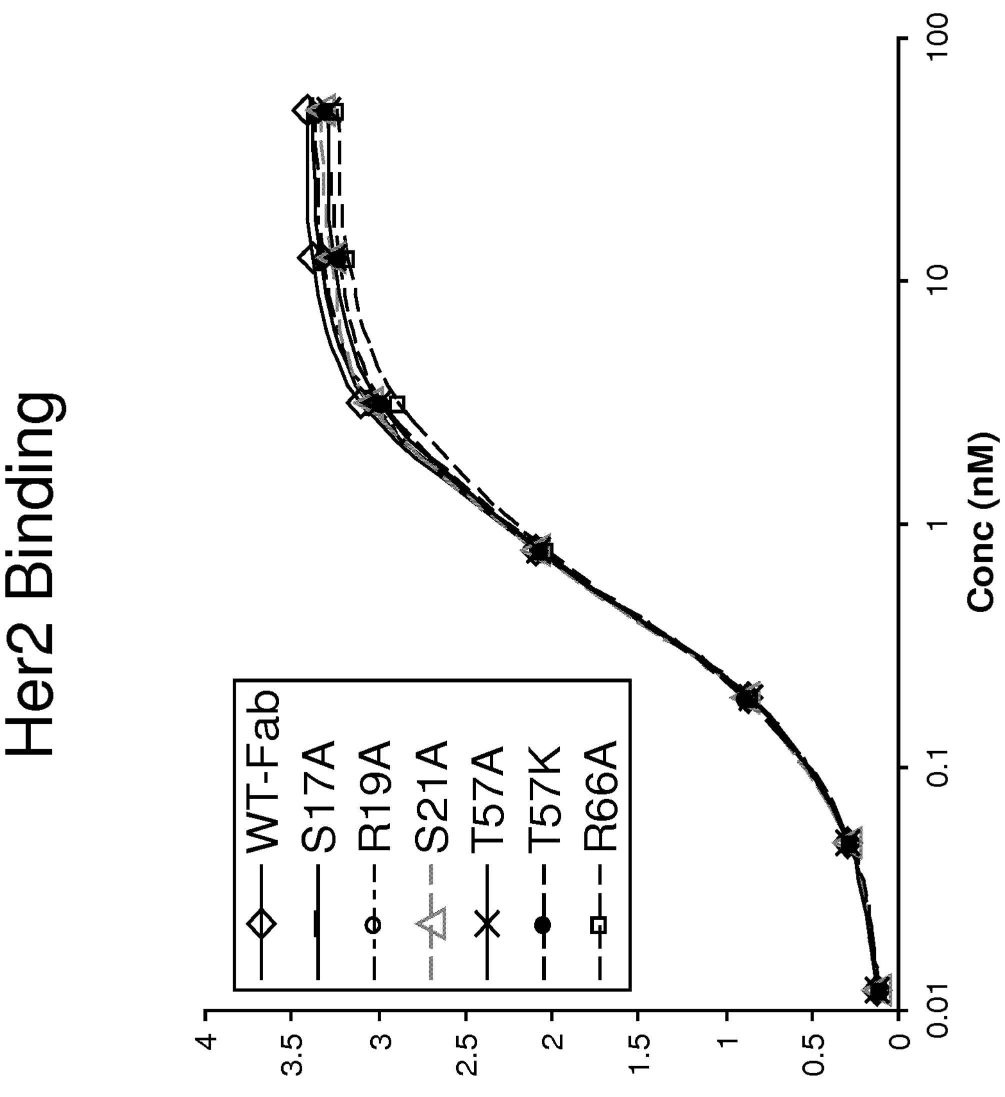
Her2 Binding
WT-Fab
S17A
R19A
S21A
T57A
T57K
R66A
0
0.5
1
1.5
2
2.5
3
3.5
4
0.01
0.1
1
10
100
Conc (nM)

Figure 1

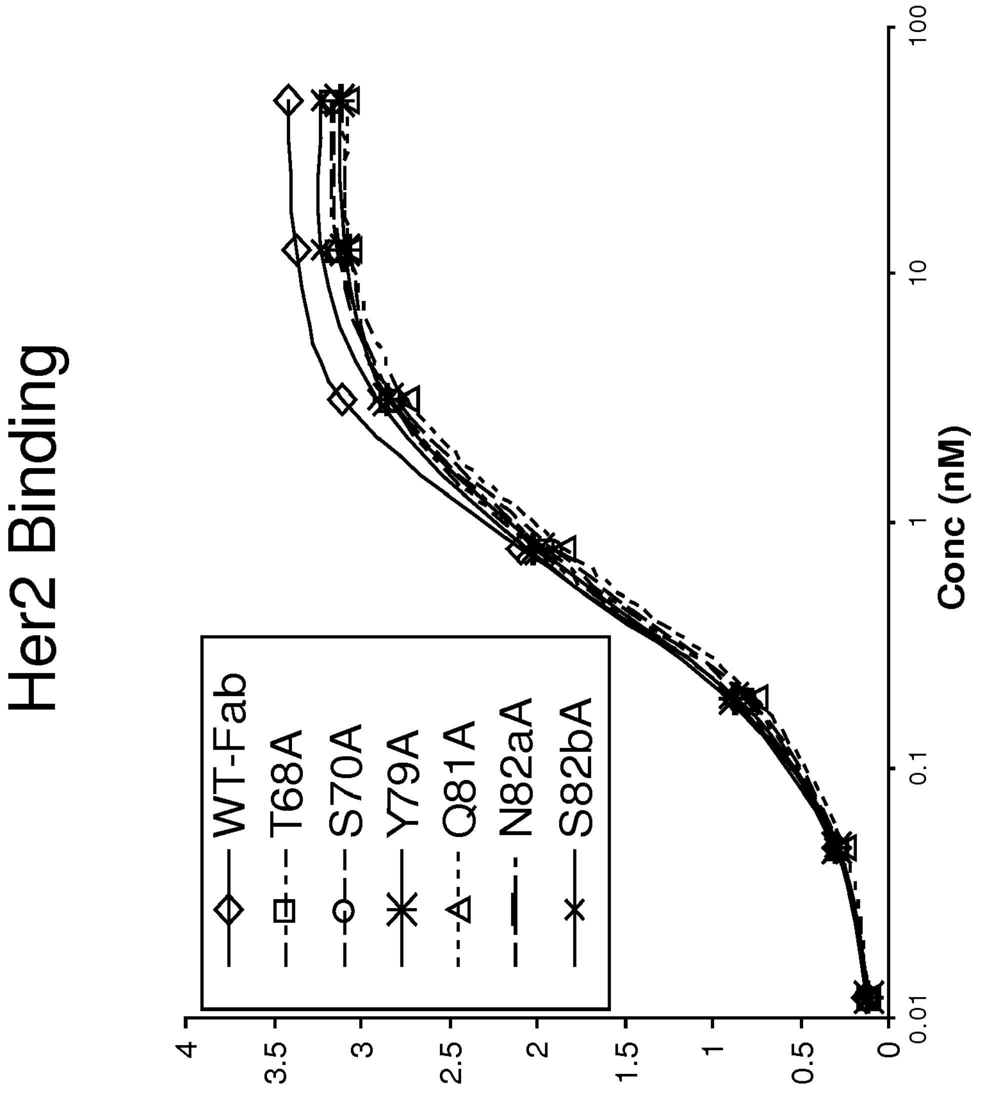
Her2 Binding
WT-Fab
T68A
S70A
Y79A
Q81A
N82aA
S82bA
4
3.5
3
2.5
2
1.5
1
0.5
0
0.01
0.1
1
10
100
Conc (nM)

Figure 2

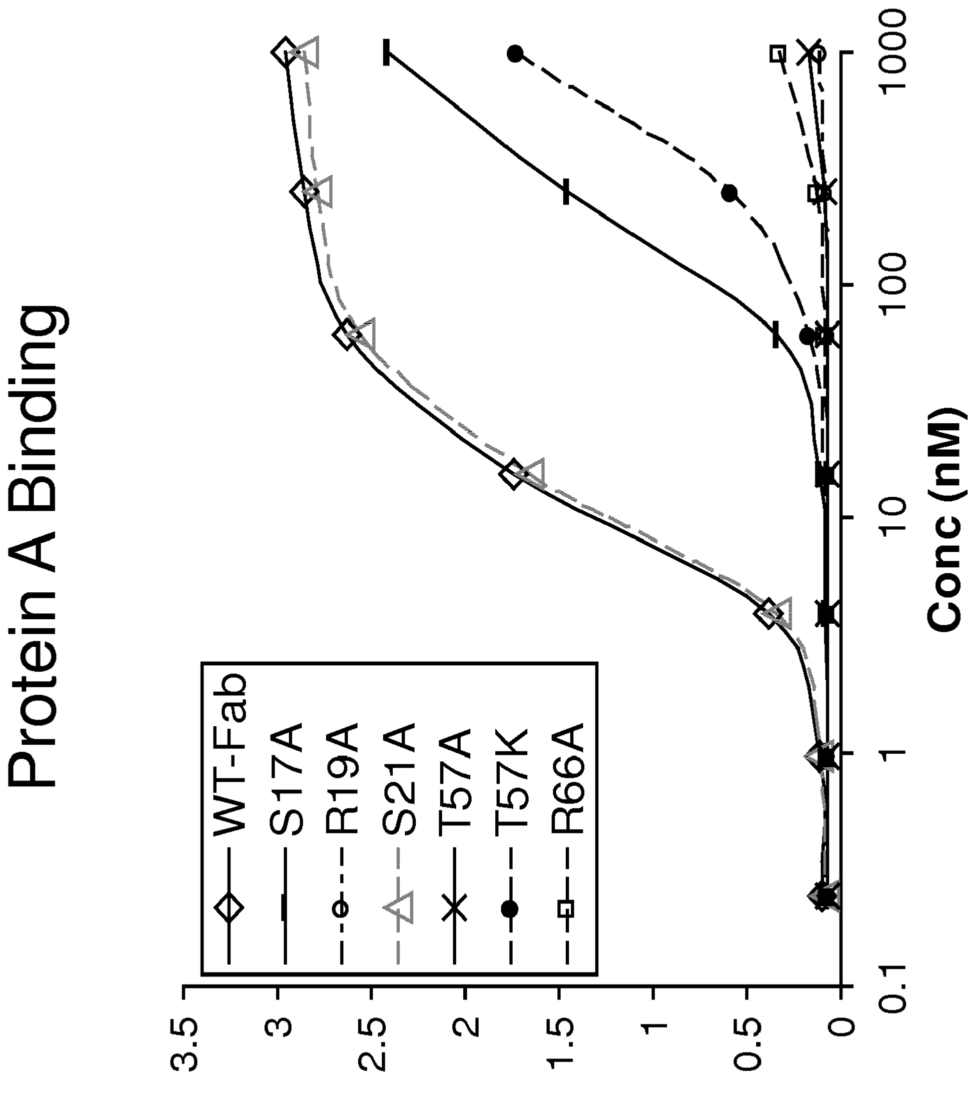
Protein A Binding
WT-Fab
S17A
R19A
S21A
T57A
T57K
R66A
3.5
3
2.5
2
1.5
1
0.5
0
0.1
1
10
100
1000
Conc (nM)

Figure 3

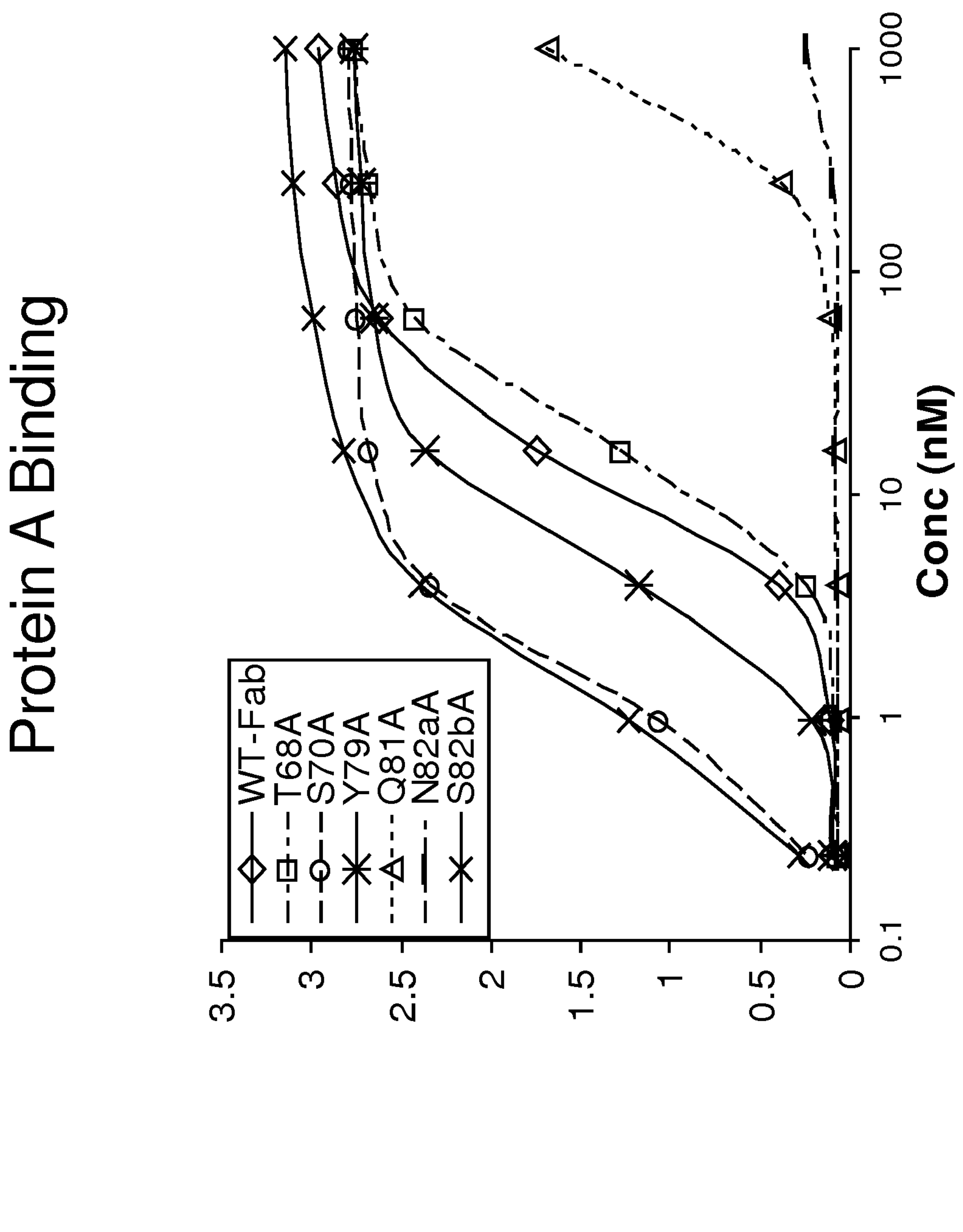
Protein A Binding
WT-Fab
T68A
S70A
Y79A
Q81A
N82aA
S82bA
3.5
3
2.5
2
1.5
1
0.5
0
0.1
1
10
100
1000
Conc (nM)

Figure 4

Europäisches Patentamt
European Patent Office
Office européen des brevets

# EUROPEAN SEARCH REPORT

Application Number
EP 17 20 2575

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 94/04679 A1 (GENENTECH INC [US]) 3 March 1994 (1994-03-03) * figure 1B; table 3; sequence 2 * | 1-12 | INV. C07K14/31 C07K16/12 A61K39/395 C07K16/00 C07K16/32 |
| A | SASSO E H ET AL: "HUMAN IGA AND IGG FAB'-2 THAT BIND TO STAPHYLOCOCCAL PROTEIN A BELONG TO THE V-HIII SUBGROUP", JOURNAL OF IMMUNOLOGY, vol. 147, no. 6, 1991, pages 1877-1883, XP002588280, ISSN: 0022-1767 | 1-12 | |
| A | RIECHMANN L ET AL: "BACKBONE ASSIGNMENT, SECONDARY STRUCTURE AND PROTEIN A BINDING OF AN ISOLATED, HUMAN ANTIBODY VH DOMAIN", JOURNAL OF BIOMOLECULAR NMR, ESCOM, LEIDEN, NL LNKD- DOI:10.1007/BF00211778, vol. 6, no. 2, 1 September 1995 (1995-09-01), pages 141-152, XP001095927, ISSN: 0925-2738 | 1-12 | TECHNICAL FIELDS SEARCHED (IPC) |
| A | TRAMONTANO A ET AL: "FRAMEWORK RESIDUE 71 IS A MAJOR DETERMINANT OF THE POSITION AND CONFORMATION OF THE SECOND HYPERVARIABLE REGION IN THE VH DOMAINS OF IMMUNOGLOBULINS", JOURNAL OF MOLECULAR BIOLOGY, LONDON, GB, vol. 215, no. 1, 5 September 1990 (1990-09-05), pages 175-182, XP002906214, ISSN: 0022-2836 | 1-12 | C07K A61K |

-/--

The present search report has been drawn up for all claims

2

| Place of search | Date of completion of the search | Examiner |
|---|---|---|
| Munich | 19 January 2018 | Turri, Matteo |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 17 20 2575

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CHOTHIA C ET AL: "CONFORMATIONS OF IMMUNOGLOBULIN HYPERVARIABLE REGIONS", NATURE, NATURE PUBLISHING GROUP, LONDON, GB LNKD- DOI:10.1038/342877A0, vol. 342, no. 6252, 21 December 1989 (1989-12-21), pages 877-883, XP002030586, ISSN: 0028-0836<br>----- | 1-12 | |
| A | GRAILLE M ET AL: "Crystal structure of a Staphylococcus aureus protein A domain complexed with the Fab fragment of a human IgM antibody: Structural basis for recognition of B-cell receptors and superantigen activity", PROCEEDINGS NATIONAL ACADEMY OF SCIENCES PNAS, NATIONAL ACADEMY OF SCIENCES, US, vol. 97, no. 10, 9 May 2000 (2000-05-09), pages 5399-5404, XP002284947, ISSN: 0027-8424, DOI: 10.1073/PNAS.97.10.5399<br>* table 1 *<br>----- | 1-12 | TECHNICAL FIELDS SEARCHED (IPC) |
| A | B. A. GALITSKY ET AL: "Predicting amino acid sequences of the antibody human VH chains from its first several residues", PROCEEDINGS NATIONAL ACADEMY OF SCIENCES PNAS, vol. 95, no. 9, 28 April 1998 (1998-04-28), pages 5193-5198, XP055374309, US<br>ISSN: 0027-8424, DOI: 10.1073/pnas.95.9.5193<br>----- | 1-12 | |

The present search report has been drawn up for all claims

2

| Place of search | Date of completion of the search | Examiner |
|---|---|---|
| Munich | 19 January 2018 | Turri, Matteo |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## ANNEX TO THE EUROPEAN SEARCH REPORT ON EUROPEAN PATENT APPLICATION NO. EP 17 20 2575

This annex lists the patent family members relating to the patent documents cited in the above-mentioned European search report.
The members are as contained in the European Patent Office EDP file on
The European Patent Office is in no way liable for these particulars which are merely given for the purpose of information.

19-01-2018

| Patent document cited in search report | Publication date | Patent family member(s) | Publication date |
|---|---|---|---|
| WO 9404679 A1 | 03-03-1994 | US 6054297 A | 25-04-2000 |
| | | WO 9404679 A1 | 03-03-1994 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 61140565 B
- WO 9322332 A
- US 6586207 B
- WO 9848032 A
- WO 03073238 A
- US 20040214988 A1
- WO 05135727 A2
- WO 0574524 A2
- EP 404097 A
- WO 199301161 A
- US 4816567 A
- WO 199824893 A
- WO 199634096 A
- WO 199633735 A
- WO 199110741 A
- US 5545807 A
- US 5545806 A
- US 5569825 A
- US 5625126 A
- US 5633425 A
- US 5661016 A
- US 6982321 B
- US 7087409 B
- US 6075181 A
- US 6150584 A
- WO 2006073941 A
- US 6194551 B1
- WO 199951642 A
- US 5641870 A
- WO 9306213 A
- WO 9308829 A
- WO 9404690 A
- US 4676980 A
- WO 9100360 A
- WO 9200373 A
- EP 03089 A
- US 20030157108 A
- US 20040093621 A
- WO 200061739 A
- WO 200129246 A
- US 20030115614 A
- US 20020164328 A
- US 20040132140 A
- US 20040110704 A
- US 20040110282 A
- US 20040109865 A
- WO 2003085119 A
- WO 2003084570 A
- WO 2005035586 A
- WO 2005035778 A
- WO 2005053742 A
- WO 2002031140 A
- US 20030157108 A1, Presta, L
- WO 2004056312 A1, Adams
- WO 2003085107 A
- WO 2003011878 A, Jean-Mairet
- US 6602684 B, Umaña
- US 20050123546 A, Umaña
- WO 199730087 A, Patel
- WO 199858964 A, Raju, S.
- WO 199922764 A, Raju, S.
- US 5500362 A
- WO 5821337 A
- US 5731168 A
- US 20020038086 A
- US 5112596 A
- US 5268164 A
- US 5506206 A
- US 5686416 A
- US 20030083299 A
- US 20020025313 A
- US 20040204354 A
- US 20040131692 A
- US 20020065259 A
- US 20030162695 A
- US 20050124533 A
- US 20050089473 A
- US 20030073713 A
- US 20030129186 A
- US 5004697 A
- US 3773919 A
- US 5821337 A


### Non-patent literature cited in the description


- **RIECHMANN ; DAVIES.** *J. Biomolecular NMR,* 1995, vol. 6, 141-52
- **ARTANDI et al.** *Proc. Natl. Acad. Sci. USA,* 1992, vol. 89, 94-98
- **SAMBROOK et al.** Molecular Cloning: A Laboratory Manual. Cold Spring Harbor Laboratory Press, 2001

- CURRENT PROTOCOLS IN MOLECULAR BIOLOGY. 2003
- METHODS IN ENZYMOLOGY. Academic Press, Inc,
- A PRACTICAL APPROACH. 1995
- ANTIBODIES, A LABORATORY MANUAL. 1988
- ANIMAL CELL CULTURE. 1987
- Oligonucleotide Synthesis. 1984
- Methods in Molecular Biology. Humana Press
- Cell Biology: A Laboratory Notebook. Academic Press, 1998
- Animal Cell Culture. 1987
- **J. P. MATHER ; P. E. ROBERTS.** Introduction to Cell and Tissue Culture. Plenum Press, 1998
- Cell and Tissue Culture: Laboratory Procedures. J. Wiley and Sons, August 1993
- Handbook of Experimental Immunology
- Gene Transfer Vectors for Mammalian Cells. 1987
- PCR: The Polymerase Chain Reaction. 1994
- Current Protocols in Immunology. 1991
- Short Protocols in Molecular Biology. Wiley and Sons, 1999
- **C. A. JANEWAY ; P. TRAVERS.** *Immunobiology,* 1997
- **P. FINCH.** *Antibodies,* 1997
- Antibodies: A Practical Approach. IRL Press, 1988
- Monoclonal Antibodies: A Practical Approach. Oxford University Press, 2000
- **E. HARLOW ; D. LANE.** Using Antibodies: A Laboratory Manual. Cold Spring Harbor Laboratory Press, 1999
- The Antibodies. Harwood Academic Publishers, 1995
- Cancer: Principles and Practice of Oncology. J.B. Lippincott Company, 1993
- **SINGLETON et al.** Dictionary of Microbiology and Molecular Biology. J. Wiley & Sons, 1994
- **MARCH.** Advanced Organic Chemistry Reactions, Mechanisms and Structure. John Wiley & Sons, 1992
- **KABAT et al.** Sequences of Proteins of Immunological Interest. Public Health Service, National Institutes of Health, 1991
- **ELLMAN et al.** *Meth. Enzym.,* 1991, vol. 202, 301-336
- **J. W. CHIN et al.** *Journal of the American Chemical Society,* 2002, vol. 124, 9026-9027
- **J. W. CHIN ; P. G. SCHULTZ.** *ChemBioChem,* 2002, vol. 11, 1135-1137
- **J. W. CHIN et al.** *PICAS United States of America,* 2002, vol. 99, 11020-11024
- **KABAT et al.** Sequences of Proteins of Immunological Interest. National Institute of Health, 1991
- **ABBAS et al.** Cellular and Mol. Immunology. W.B. Saunders, Co, 2000
- **PLUCKTHÜN.** The Pharmacology of Monoclonal Antibodies. Springer-Verlag, 1994, vol. 113, 269-315
- **HUDSON et al.** *Nat. Med.,* 2003, vol. 9, 129-134
- **HOLLINGER et al.** *Proc. Natl. Acad. Sci. USA,* 1993, vol. 90, 6444-6448
- **KOHLER ; MILSTEIN.** *Nature,* 1975, vol. 256, 495-97
- **HONGO et al.** *Hybridoma,* 1995, vol. 14 (3), 253-260
- **HARLOW et al.** Antibodies: A Laboratory Manual. Cold Spring Harbor Laboratory Press, 1988
- **HAMMERLING et al.** Monoclonal Antibodies and T-Cell Hybridomas. Elsevier, 1981, 563-681
- **CLACKSON et al.** *Nature,* 1991, vol. 352, 624-628
- **MARKS et al.** *J. Mol. Biol.,* 1992, vol. 222, 581-597
- **SIDHU et al.** *J. Mol. Biol.,* 2004, vol. 338 (2), 299-310
- **LEE et al.** *J. Mol. Biol.,* 2004, vol. 340 (5), 1073-1093
- **FELLOUSE.** *Proc. Natl. Acad. Sci. USA,* 2004, vol. 101 (34), 12467-12472
- **LEE et al.** *J. Immunol. Methods,* 2004, vol. 284 (1-2), 119-132
- **JAKOBOVITS et al.** *Proc. Natl. Acad. Sci. USA,* 1993, vol. 90, 2551
- **JAKOBOVITS et al.** *Nature,* 1993, vol. 362, 255-258
- **BRUGGEMANN et al.** *Year in Immunol.,* 1993, vol. 7, 33
- **MARKS et al.** *Bio/Technology,* 1992, vol. 10, 779-783
- **LONBERG et al.** *Nature,* 1994, vol. 368, 856-859
- **MORRISON.** *Nature,* 1994, vol. 368, 812-813
- **FISHWILD et al.** *Nature Biotechnol.,* 1996, vol. 14, 845-851
- **NEUBERGER.** *Nature Biotechnol.,* 1996, vol. 14, 826
- **LONBERG ; HUSZAR.** *Intern. Rev. Immunol.,* 1995, vol. 13, 65-93
- **MORRISON et al.** *Proc. Natl. Acad. Sci.,* 1984, vol. 81, 6851-6855
- **JONES et al.** *Nature,* 1986, vol. 321, 522-525
- **RIECHMANN et al.** *Nature,* 1988, vol. 332, 323-329
- **PRESTA.** *Curr. Op. Struct. Biol.,* 1992, vol. 2, 593-596
- **VASWANI ; HAMILTON.** *Ann. Allergy, Asthma & Immunol.,* 1998, vol. 1, 105-115
- **HARRIS.** *Biochem. Soc. Transactions,* 1995, vol. 23, 1035-1038
- **HURLE ; GROSS.** *Curr. Op. Biotech.,* 1994, vol. 5, 428-433

- **HOOGENBOOM ; WINTER.** *J. Mol. Biol.,* 1991, vol. 227, 381
- **MARKS et al.** *J. Mol. Biol.,* 1991, vol. 222, 581
- **COLE et al.** Monoclonal Antibodies and Cancer Therapy. Alan R. Liss, 1985, 77
- **BOERNER et al.** *J. Immunol.,* 1991, vol. 147 (1), 86-95
- **VAN DIJK ; VAN DE WINKEL.** *Curr. Opin. Pharmacol.,* 2001, vol. 5, 368-74
- **LI et al.** *Proc. Natl. Acad. Sci.,* 2006, vol. 103, 3557-3562
- **XU et al.** *Immunity,* 2000, vol. 13, 37-45
- **JOHNSON ; WU.** Methods in Molecular Biology. Human Press, 2003, vol. 248, 1-25
- **HAMERS-CASTERMAN et al.** *Nature,* 1993, vol. 363, 446-448
- **SHERIFF et al.** *Nature Struct. Biol.,* 1996, vol. 3, 733-736
- **CHOTHIA ; LESK.** *J. Mol. Biol.,* 1987, vol. 196, 901-917
- **KABAT et al.** Sequences of Immunological Interest. Public Health Service, National Institutes of Health, 1991
- **BARBAS et al.** *Proc Nat. Acad. Sci.,* 1994, vol. 91, 3809-3813
- **SCHIER et al.** *Gene,* 1995, vol. 169, 147-155
- **YELTON et al.** *J. Immunol.,* 1995, vol. 155, 1994-2004
- **JACKSON et al.** *J. Immunol.,* 1995, vol. 154 (7), 3310-9
- **HAWKINS et al.** *J. Mol. Biol.,* 1992, vol. 226, 889-896
- **BURTON.** *Molec. Immunol.,* 1985, vol. 22, 161-206
- **DAËRON.** *Annu. Rev. Immunol.,* 1997, vol. 15, 203-234
- **RAVETCH ; KINET.** *Annu. Rev. Immunol,* 1991, vol. 9, 457-92
- **CAPEL et al.** *Immunomethods,* 1994, vol. 4, 25-34
- **DE HAAS et al.** *J. Lab. Clin. Med.,* 1995, vol. 126, 330-41
- **GAZZANO-SANTORO et al.** *J. Immunol. Methods,* 1996, vol. 202, 163
- **IDUSOGIE et al.** *J. Immunol.,* 2000, vol. 164, 4178-4184
- **CHEN et al.** *J. Mol. Biol.,* 1999, vol. 293, 865-881
- **KNAPPEK et al.** *J. Mol. Biol.,* 1999, vol. 296, 57-86
- **GARRARD ; HENNER.** *Gene,* 1993, vol. 128, 103
- **ZAPATA et al.** *Protein Eng,* 1995, vol. 8 (10), 1057-1062
- **M.-P. LEFRANC ; G. LEFRANC.** The Human IgG Subclasses. Pergamon Press, 1990, 43-78
- **MORIMOTO et al.** *Journal of Biochemical and Biophysical Methods,* 1992, vol. 24, 107-117
- **BRENNAN et al.** *Science,* 1985, vol. 229, 81
- **CARTER et al.** *Bio/Technology,* 1992, vol. 10, 163-167
- **RIECHMANN et al.** *Nature,* 1988, vol. 332, 323-327
- **VERHOEYEN et al.** *Science,* 1988, vol. 239, 1534-1536
- **SIMS et al.** *J. Immunol.,* 1993, vol. 151, 2296
- **CHOTHIA et al.** *J. Mol. Biol.,* 1987, vol. 196, 901
- **CARTER et al.** *Proc. Natl. Acad. Sci. USA,* 1992, vol. 89, 4285
- **PRESTA et al.** *J. Immunol.,* 1993, vol. 151, 2623
- **KOZBOR.** *J. Immunol.,* 1984, vol. 133, 3001
- **BRODEUR et al.** Monoclonal Antibody Production Techniques and Applications. Marcel Dekker, Inc, 1987, 51-63
- **BOERNER et al.** *J. Immunol.,* 1991, vol. 147, 86
- **JAKOBOVITS et al.** *Proc. Natl. Acad. Sci USA,* 1993, vol. 90, 2551
- **JAKOBOVITS et al.** *Nature,* 1993, vol. 362, 255
- **BRUGGERMANN et al.** *Year in Immunol.,* 1993, vol. 7, 33
- **MILSTEIN ; CUELLO.** *Nature,* 1983, vol. 305, 537
- **TRAUNECKER et al.** *EMBO J.,* 1991, vol. 10, 3655
- **SURESH et al.** *Methods in Enzymology,* 1986, vol. 121, 210
- **GRUBER et al.** *J. Immunol.,* 1994, vol. 152, 5368
- **TUTT et al.** *J. Immunol.,* 1991, vol. 147, 60
- **CUNNINGHAM ; WELLS.** *Science,* 1989, vol. 244, 1081-1085
- **WRIGHT et al.** *TIBTECH,* 1997, vol. 15, 26-32
- **OKAZAKI et al.** *J. Mol. Biol.,* 2004, vol. 336, 1239-1249
- **YAMANE-OHNUKI et al.** *Biotech. Bioeng.,* 2004, vol. 87, 614
- **RIPKA et al.** *Arch. Biochem. Biophys.,* 1986, vol. 249, 533-545
- **KANDA, Y. et al.** *Biotechnol. Bioeng.,* 2006, vol. 94 (4), 680-688
- **RAVETCH ; KINET.** *Annu. Rev. Immunol.,* 1991, vol. 9, 457-92
- **HELLSTROM, I. et al.** *Proc. Nat'l Acad. Sci.,* 1986, vol. 83, 7059-7063
- **HELLSTROM, I et al.** *Proc. Nat'l Acad. Sci. USA,* 1985, vol. 82, 1499-1502
- **BRUGGEMANN, M. et al.** *J. Exp. Med.,* 1987, vol. 166, 1351-1361
- **CLYNES et al.** *Proc. Nat'l Acad. Sci. USA,* 1998, vol. 95, 652-656
- **CRAGG, M.S. et al.** *Blood,* 2003, vol. 101, 1045-1052

- **CRAGG, M.S. ; M.J. GLENNIE.** *Blood,* 2004, vol. 103, 2738-2743
- **PETKOVA, S.B. et al.** *Int'l. Immunol.,* 2006, vol. 18 (12), 1759-1769
- **A. L. LEHNINGER.** Biochemistry. Worth Publishers, 1975, 73-75
- **KAM et al.** *Proc. Natl. Acad. Sci. USA,* 2005, vol. 102, 11600-11605
- **MANIATIS.** Molecular Cloning--A Laboratory Manual. Cold Spring Harbor Laboratory Press, 2001
- Current Protocols in Molecular Biology. John Wiley & Sons
- Antibody Purification: Principles and Practice. Springer-Verlag, 1994
- **PAPANASTASSIOU et al.** *Gene Therapy,* 2002, vol. 9, 398-406
- **BOBO et al.** *Proc. Natl. Acad. Sci. USA,* 1994, vol. 91, 2076-2080
- **GILL et al.** *Nature Med.,* 2003, vol. 9, 589-595
- **NEUWELT, E. A.** Implication of the Blood-Brain Barrier and its Manipulation. Plenum Press, 1989, vol. 1 & 2
- **BEHRSTOCK et al.** *Gene Ther.,* 15 December 2005
- Remington: The Science and Practice of Pharmacy. 2000